# EUROPEAN PATENT APPLICATION

(11) **EP 3 144 007 A1**
(43) Date of publication of application: **22.03.2017**
(21) Application number: 15186045.9
(22) Date of filing: 21.09.2015
(51) Int. Cl.: A61K 38/00

(54) **PEPTIDE FOR USE IN THE TREATMENT OF DISEASE CAUSED BY CLOSTRIDIUM NEUROTOXINS**

(71) Applicant: Universitat Autònoma de Barcelona, 08193 Bellaterra (Cerdanyola del Valles) (ES); Benemérita Universidad Autónoma de Puebla, 72000 Puebla (MX)
(72) Inventor: Aguiilera Ávila, José, 08912 Badalona (ES); Candalija Iserte, Ana María, 08921 Santa Coloma de Gramenet (ES); Scior, Thomas Rainer Friedrich, 72000 Puebla, México (DE)
(74) Representative: Vallvé Sánchez, Xavier

(57) **Abstract**

The present invention relates to a cyclic peptide and to its use as an inhibitor of clostridium neurotoxins, preferably tetanus and botulinum neurotoxins. The peptide of the invention is useful in the treatment and/or prevention of the diseases caused by said clostridium neurotoxins. The invention also relates to the nucleic acid that codifies it, to a cell, a pharmaceutical composition and a kit that comprises it.

SEQUENCE LISTING

<110>  UNIVERSITAT AUTONOMA DE BARCELONA

<120>  PEPTIDE FOR USE IN THE TREATMENT OF DISEASE CAUSED BY CLOSTRIDIUM
       NEUROTOXINS

<130>  EP3305.2

<160>  4

<170>  PatentIn version 3.5

<210>  1
<211>  14
<212>  PRT
<213>  Artificial Sequence

<220>
<223>  peptide "first half 1"

<400>  1

Gly Cys Leu Gln Leu Asp Asn Pro Thr His Met Asn Asn Gly
1               5                   10


<210>  2
<211>  13
<212>  PRT
<213>  Artificial Sequence

<220>
<223>  peptide "second half 2"

<400>  2

Met Leu Gln Leu Asp Asn Pro Thr His Cys Asn Asn Gly
1               5                   10


<210>  3
<211>  27
<212>  PRT
<213>  Artificial Sequence

<220>
<223>  peptide "first half 1 - second half 2"

<400>  3

Gly Cys Leu Gln Leu Asp Asn Pro Thr His Met Asn Asn Gly Met Leu
1               5                   10                  15


Gln Leu Asp Asn Pro Thr His Cys Asn Asn Gly
            20                  25


<210>  4
<211>  17
<212>  PRT
<213>  Artificial Sequence

<220>
<223>  scrambled peptide

<400>  4

Ser Ala Pro Ala Thr Gly Gly Val Lys Lys Pro His Arg Tyr Arg Pro
1               5                   10                  15

Gly

## Description

The invention relates to a cyclic peptide that inhibits clostridium neurotoxins, and therefore is useful in the prophylaxis and/or treatment of the diseases caused by these neurotoxins. The invention can be circumscribed to the Medicine and Veterinarian fields.

### BACKGROUND ART

Clostridium neurotoxins present a threat both to humans and animals. Clostridium neurotoxins can be life-threatening in subnanomolar range acting on human nerve system cells after entering the nervous system at the neuromuscular junctions [Gill, 1982]. The seven immunologically distinct serotypes (A, B, C1, D, E, F and G) of botulinum neurotoxin (BoNT) as well as tetanus toxin are proteins produced by *Clostridium botulinum* or *Clostridium tetani,* respectively. In particular, four serotypes A, B, E, and F are responsible for the botulism disease [Zhang *et al.,* 2011]. Both infections hit the nerve system on a molecular level and clinically lead to sometimes - deadly botulism and tetanus diseases, respectively.

In the case of the botulinum disease (botulism) the source of infection is food intake and the following lethal intoxication is caused by the toxic protein, called botulinum neurotoxin (BoNT). It is produced in anaerobic conditions, contaminating mostly canned food or especially meat, which is then not sufficiently boiled or cored before consuming the contaminated food. Like *Clostridium tetani,* the other pathogenic bacterium of the *Clostridium* family, *Clostridium botulinum* is a large anaerobic Gram-positive bacillus. Botulinum neurotoxin is broken into 7 neurotoxins (labelled as types A, B, C1, D, E, F, and G), which are antigenically and serologically distinct but structurally similar. Others species of Clostridium as *C*. *butyricum* and *C*. *baratii* produce BoNT/E, and *C*. *baratii* and *C*. *argentinense* produce BoNT/F. Human botulism is caused mainly by types A, B, E, and (rarely) F. Types C1 and D cause toxicity only in animals [Childers, M., 2012].

If diagnosed early, foodborne and wound botulism can be treated by inducing passive immunity with a horse-derived antitoxin, which blocks the action of toxin circulating in the blood [Shapiro *et al.,* 1998]. Food-borne botulism can lead to severe respiratory failure and paralysis. The only drug currently available to treat infant botulism is intravenously applied human botulism immune globulin [Brook, 2007]. There are two primary botulinum antitoxins available for treatment of wound and foodborne botulism. Trivalent (A, B, E) botulinum antitoxin is derived from equine sources utilizing whole antibodies (Fab & Fc portions). A second-generation vaccine has a multivalent antitoxin against all seven BoNT serotypes A, B, C1, D, E, F and G. There is also a heptavalent vaccine derived from horse Immunoglobulin G antibodies which have had the Fc portion cleaved off leaving only the F(ab')2 portions, in general, it is a less immunogenic antitoxin but works against all known pathogenic strains of botulism [Smith and Rusnak, 2007]. Other strategies to protect from botulinum infections were reviewed very recently [Wilson and Ho, 2014]. One of the strategies is the development to target the toxicity of clostridial bacteria by inactivating their zinc-proteases with drug-like substances. But, to date, they did not succeed and no adequate substances could be found as potential solutions against the clostridial neurotoxins in living organisms [Supuran, 2012]. Another anti-microbial strategy consists in the application of bacteriocines, which were discovered in *Escherichia coli* and first described in the second decade of the XX century. They are proteins, which appear excreted outside the living bacteria cells for protection and growth inhibition of other bacteria in the neighborhood; some also act in case of botulinum as bio-conservatives [Cui *et al.,* 2012].

In farm animals botulinum associated diseases are also increasing. For instance, glyphosate, an herbicide found in cattle livestock could be associated with the increase in *C*. *botulinum* mediated diseases in cows. Tetanus disease is caused by a bacterial protein called tetanus toxin (TeTx or TeNT). It is neurotoxic and lethal for untreated patients without tetanus vaccination. The TeNT protein is produced by bacteria and called *Clostridium tetani.* The bacteria live under anaerobic conditions in mud, dirty surfaces and earthen media etc., and enter the body through open wounds.

*C. tetani* bacteria usually enter the body through a wound while *C*. *botulinum* shows also this route on occasions. In the presence of anaerobic (lacking oxygen) conditions, the spores germinate. Especially, tetanus toxins are produced and disseminated via blood or lymphatic systems. The toxins interact at several sites within the central nervous system, including peripheral motor end plates, spinal cord, brain, and in the sympathetic nervous system. The typical clinical manifestations of tetanus are caused when tetanus toxin interferes with release of neurotransmitters, blocking principally inhibitor impulses. This leads to unopposed muscle contraction and spasm. Seizures may occur, and the autonomic nervous system may also be affected.

Tetanus infection requires intensive medical assistance and hospitalization of the patient. Human tetanus immune globulin (TIG) or antitoxin form horse (equine immune globulins) is administered as soon as possible as causal therapy. For the treatment of other symptoms like muscle spasms or wound infections, muscle relaxing drugs and antibiotics are administered as co-medication. In case of respiratory failure (chest spasm), breathing is assisted by artificial respiration in intensive care units [Hassel, 2013].

Tetanus vaccine contains a non-pathogenic form of the tetanus toxin, inactivated, non-toxic protein, and causes an immune response, which includes the production of antibodies. As with any medications, there are side effects to these vaccines.

TeNT and, some of the seven BoNT serotypes different neurotoxins, bind to the same cell membrane receptors, which are the molecular target structures of known neurotrophins, i.e. the cell membrane receptors of neurotrophins (NTRs). The common binding site of NTRs is the ectodomain d5 of TrkA, TrkB and TrkC on the cell surface. It is occupied by neurotrophins under physiological conditions or neurotoxins in cases of clostridial infections.

Despite the efforts made in the treatment of these intoxications, there is an urgent need of an antidote against tetanus and botulinum that could act during an early stage, neutralizing the toxins by impairing its binding to their receptors.

### SUMMARY OF THE INVENTION

The present invention discloses a novel antidote for clostridium neurotoxins, particularly to tetanus toxin and botulinum neurotoxin serotypes A, B, C1, D, E, F and G which inhibits the binding of those toxins to their specific receptors, particularly to TrkB, TrkA, TrkC and with different strategic p75^{NTR}. This novel antidote is a cyclic peptide (but could also work as a lineal peptide, therefore the present invention also refers to the linear peptide of the invention) that comprises an amino acid sequence of at least 70% of identity with sequence SEQ ID NO: 1, preferably the peptide is SEQ ID NO: 1. The present invention demonstrates the inhibition of those neurotoxins by the exemplification with the cyclic peptide of sequence SEQ ID NO: 3.

The cyclic peptide of the invention is a non-naturally occurring peptide. The peptide is based in a portion of TrkB.

The cyclic peptide of the invention replaces the common binding site of TrkB, TrkA and TrkC (the d5 domain) and thereby neutralizes clostridial neurotoxins, leaving the common binding site of NTRs accessible to their physiological binders: the neurotrophins. The therapeutic presentation of the antidote of the present invention as a "false receptor" (mimetic, suicide molecule or reverse-decoy) "neutralizes" incoming botulinum toxin (BoNT) and tetanus toxin (also called spasmogenic toxin, tetanospasmin or tetanus neurotoxin, in short TeNT or TeTx). The intoxication process is effectively stopped at this early stage, preventing deeper invasion and further cell damage. Mutational studies were successfully carried out as proof of concept in which amino acids where changed to show that they play a role at the binding site between bacterial toxins and the cell surface receptor prior to cell uptake.

The antidote can be administered in infected or intoxicated patients, even with an impaired immune status, or without tetanus vaccination or in individuals or unknown status of vaccination.

The antidote of the invention can be applied as an emergency remedy in humans and animals, for example administered in bolus or infusion dosage forms under parenteral, i.e. intra-venous administration.

In the present invention, computer-based (*in silico*) research work is shown and *also in vitro* experiments demonstrating the usefulness of the peptide of the invention.

The term "Clostridial infections", as used herein, refers to aforementioned both infections caused by clostridium neurotoxins, which are proteins produced by *C*. *botulinum* (in a few cases *C baratii, C. argentinense* or *C*. *butiricum*) or *C*. *tetani.*

A first aspect of the present invention relates to a cyclic peptide that comprises an amino acid sequence of at least 70%, 80%, 85%, 90%, 95%, 96%, 97%, 98%, 99% or 100% identity with sequence SEQ ID NO: 1 (GCLQLDNPTHMNNG) preferably the peptide is SEQ ID NO: 1. From now on, this peptide is named as the "peptide of the invention" or the "cyclic peptide of the invention". This is also describes as "the antidote" in the present invention.

The term "cycled" is used herein to refer to a close structure or a molecular "ring".

In the present invention the peptide of the invention is also named as "d5 ring" or as "d5 peptide" in short - whereas the entire ectodomain d5 of Trk NTRs is also referred to as "d5" in short.

The term "identity", as used herein, refers to the percentage of identical amino acid residues between two peptides when they are compared. The sequence comparison methods are known in the art, and include but are not limited to, the BLASTP or BLASTN, and FASTA program ClustalW. The expert in the field can consider that peptides identity percentages of at least 70%, 80%, 85%, 90%, 95%, 96%, 97%, 98% or 99% maintain the same properties and functions of said polypeptide (the functions indicated in this description, therefore, as inhibitor of a clostridium neurotoxin, preferably the clostridium neurotoxin is selected from the list consisting of: tetanus toxin, botulinum toxin A, botulinum toxin B, botulinum toxin C1, botulinum toxin D, botulinum toxin E, botulinum toxin F, botulinum toxin G, and any combinations thereof).

The percentage of identity in the present invention is of at least 70%; therefore, it can be 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100%.

A preferred embodiment of the first aspect of the present invention refers to the cyclic peptide wherein it also comprises at least another amino acid sequence (preferably just one) of at least 70% of identity with sequence SEQ ID NO: 1, preferably amino acid sequence SEQ ID NO: 2 (MLQLDNPTHCNNG).

In another preferred embodiment of this first aspect of the invention, SEQ ID NO: 2 is joined to the carboxyl-terminal end of the SEQ ID NO: 1 in order to give rise to a fusion peptide. This bind may be direct or by means of a linker polypeptide.

The term "linker polypeptide" or "linker" as used in the present description refers to a short amino acid sequence, preferably, of up to 20 amino acids in length, situated between the sequences of amino acids.

In another preferred embodiment of the first aspect of the present invention the cyclic peptide comprises sequence SEQ ID NO: 3. In a more preferred embodiment the cyclic peptide consists of sequence SEQ ID NO: 3 (GCLQLDNPTHMNNGMLQLDNPTHCNNG) (also named as "first half 1 - second half 2").

The present invention also refers to the linear peptide of the invention, preferably consisting of SEQ ID NO: 1, SEQ ID NO: 2 or SEQ ID NO: 3.

The cyclic peptide of the first aspect of the invention is preferably cycled between at least two cysteine amino acids forming a disulfide bridge, for example, disulfide bridges by "head to tail" cyclization.

In a preferred embodiment the cycle in SEQ ID NO: 3 is located between the position 2 and 24 between the two cysteines.

Other types of cyclization of the peptide of this invention are possible by other methods. The resulting molecular ring has to "freeze-in" or to preserve the same or similar conformational behavior (for neurotoxin binding) as the present cyclization via a disulfide bridge of the cyclic peptide of the invention. In another preferred embodiment of the first aspect of the present invention the peptide has modifications in at least one of the terminal ends. The modifications on either end (N- or C-terminal ends), ideally, would contribute to enhance potency, hydro-solubility or metabolic stability and thereby the effective time of action (effect duration). The modifications can be additional amino acids, sugar-like residues, or chemical groups known in the art to protect peptide bonds and disulfide bridge from non-enzymatic or enzymatic cleavage.

In the peptide SEQ ID NO: 3 of the present invention the ring is formed between position 2 and 24, so amino acids in positions 1 and 25, 26, 27 remain exocyclic. Those exocyclic amino acids for example can be changed by derivatives and they can also add other moieties as outlined in the last paragraph above.

In general, those skilled in the art will appreciate that minor deletions or substitutions may be made to the amino acid sequences of peptides of the present invention without unduly adversely affecting the activity thereof. Thus, peptides containing such deletions or substitutions are a further aspect of the present invention. In peptides containing substitutions or replacements of amino acids, one or more amino acids of a peptide sequence may be replaced by one or more other amino acids wherein such replacement does not affect the function of that sequence. Such changes are known as homologous changes and they can be guided by known similarities between amino acids in physical features such as charge density, hydrophobicity/hydrophilicity, size and configuration, so that amino acids are substituted with other amino acids having essentially the same functional properties. Therefore, the present application also refers to the peptide analogues to the peptide of the invention. Changes are possible which are of homologous nature without disturbing physicochemical properties.

The term "peptide analogues of polypeptides" refers to peptides where at least one of their amino acids has been substituted by another one of similar characteristics, which is known as homology, as for example the ones indicated below:
- Polar neutral or hydrophilic: serine (Ser, S), threonine (Thr, T), cysteine (Cys, C), asparagine (Asn, N) and glutamine (Gin, Q).
- Nonpolar neutral, nonpolar or hydrophobic: glycine (Gly, G), alanine (Ala, A), valine (Val, V), leucine (Leu, L), isoleucine (Ile, I), methionine (Met, M), proline (Pro, P), phenylalanine (Phe, F), tryptophan (Trp, W) and tyrosine (Tyr, Y)
- With negative charge or acidic: aspartic acid (Asp, D) and glutamic acid (Glu, E).
- With positive charge or basic: lysine (Lys, K), arginine (Arg, R) and histidine (His, H).

All peptide sequences mentioned herein are written according to the usual convention whereby the N-terminal amino acid starts with the leftmost amino acid to the left and ends with the C-terminal amino acid to the right. Once made, changes can be routinely screened to determine their effects on the function of the corresponding peptide by screening procedures and experiments known by the expert in the field.

The peptide of the invention can be modified with chemical groups. The modifications on side chain or even backbone atoms, ideally, would contribute to enhance solubility or metabolic stability and thereby the effective time of action (effect duration). The chemical modifications can be additional single, double or triple bonds to amino acids, extra-ring closures, which are known in the art to protect bonds from non-enzymatic or enzymatic cleavage. A more illustrative than complete listing follows with common concepts of modifications: linear peptide branching, acetylation, methylation or phosphorylation of amino acids, sugar-modified residues, D-amino acids, amide groups at C-terminus, peptide-protein conjugates, peptide-fatty acid conjugates, peptide-chelator conjugates, peptide-PEG conjugates, or fluorescence markers and isotopes for screening assays.

Peptides of the present invention may be made in accordance with techniques known in the art, and can be recombinant peptides or chemically synthesized peptides. Using accepted techniques of chemical synthesis the peptide may be built up either from the N-terminus or - more typically - the C-terminus using either single amino acids or preformed peptides containing two or more amino acid residues.

Particular techniques for synthesizing peptides include classical methods in which peptides of increasing size are isolated before each amino acid or preformed peptide addition, classical chemical synthesis amino acid by amino acid, and solid phase peptide synthesis in which the peptide is built up attached to a resin such as a Merrifield resin. In these synthetic procedures, groups on the amino acids will generally be in a protected from using standard protecting groups such as t-butoxycarbonyl. If necessary, these protecting groups are cleaved once the synthesis is complete. Chemistry synthesis methods - for example by peptide synthesis in solid phase, solution synthesis, a combination of methods of solid phase synthesis and solution or enzymatic synthesis - are known by the expert in the field.

Peptides of the present invention may also be produced through recombinant DNA procedures as are known in the art. Modifications may be introduced during or after the synthesis of the peptide, for example a label attached for purification, a purification tag.

The term "purification tag" or "affinity tag" as used herein refers to an amino acid sequence which has been incorporated (in general genetically engineered) to a protein or peptide to facilitate purification (for example in affinity chromatography). Purification tags known in the prior art are for instance, but not limited to, the calmodulin-binding peptide (CBP), the enzyme glutathione-S-transferase (GST) or histidine residues (HIS-tag).

Herein the terms "amino acid sequence", "polypeptide", "peptide" and "oligopeptide" are used interchangeably.

A second aspect of the present invention relates to a nucleic acid that codifies for the cyclic peptide of the first aspect of the invention.

Herein the terms "nucleotide sequence", "polynucleotide", "nucleic acid" and "oligonucleotide" are used interchangeably.

The polynucleotides of the present invention may have conservative substitutions, known to the skilled expert. Conservative substitutions lead to the polynucleotides that encode the same peptide.

The term polynucleotide includes genomic DNA or DNA encoding double or single stranded, ribonucleic acid (RNA), any synthetic and genetically manipulated polynucleotide, and both sense and antisense strands. This includes single-chain molecules and double-stranded, such as DNA-DNA hybrids, DNA-RNA and RNA-RNA.

The nucleotide sequence of the invention can be obtained artificially by conventional cloning methods selection and widely known in the prior art.

The nucleotide sequence of the invention, in addition to the coding sequence, may have other elements, such as but not limited, introns, noncoding sequences at the 5' or 3', binding sites for ribosomes, stabilizing sequences, cell detection, etc. These polynucleotides may also include further coding sequences for additional amino acids, which may be useful, for example, but not limited, to enhance stability of the peptide generated from it or to allow a better purification thereof.

The nucleic acid can form part of an expression cassette, comprising the nucleic acid of the invention operatively linked to control elements of transcription and/or translation.

The term "operatively linked" refers to a juxtaposition in which the components herein described have a relationship that enables them to work in the intentional way. A polynucleotide operatively linked to any regulatory sequence that controls the expression of said polynucleotide is linked in such a way that the expression of the coding sequence of the polynucleotide is achieved under conditions compatibles with the regulatory sequence.

The term "regulatory sequence that controls the expression of a polynucleotide" refers to a nucleotidic sequence having an effect on the functionality of the polynucleotide gene referred to the beginning of the transcript from a DNA sequence or at the start of translation of a sequence of RNA or other sequences not described. By way of example, between regulatory sequences of the gene expression referred to in the present invention are the promoters and others less common as certain introns. The nature of these regulatory sequences differs depending on the host organism; in prokaryotes, these sequences of control usually include a promoter, a site of ribosomal union, and signs of completion; in eukaryotes, usually, these sequences of control include promoters, signs of termination, intensifiers and sometimes silencers.

As it is used here, the term "promoter" refers to a region of the DNA upstream from the start point of the transcription of a gene, and particularly, that is able to start the transcription in a cell. Promoters can be classified, for instance, as inducible and constitutive promoters.

The polynucleotide sequence described in the second aspect of the present invention may be found forming part of vectors allowing the propagation or cloning and expression. Said vector may be, for example, a cloning vector or an expression vector or recombinant vector. The cloning of the nucleotide sequence of the invention may be performed using an expression vector or recombinant vector or a plasmid. These vectors comprise the nucleotide sequence of the invention (or the expression cassette) and may also comprise a series of amino acid coding sequences and targets for protease cleavage. The advantage of this structure is that once produced the fusion protein or peptide, lysate expression system and having it purified by - for example, but not limited to - affinity chromatography, the peptide may be cleaved from the carrier protein of the invention by digestion with a protease and repurification of the peptide of the invention using the same chromatographic system.

Therefore a third aspect of the present invention relates to a vector that comprises the nucleic acid of the second aspect of the invention.

The term "vector", as used herein, refers to a nucleic acid molecule that is capable of transferring nucleic acid sequences contained therein to a cell. Examples of recombinant vectors are linear DNA, plasmid DNA, modified viruses, adenovirus/adeno-associated viruses, and retroviral viral vectors, etc.; all widely described in the literature and which can be used following standard molecular biology techniques or purchased from vendors. Among the most commonly used are viral vectors for the introduction of genes in mammalian cells such as poxvirus vectors, herpes simplex, adenovirus, adeno-associated vectors, etc.

Vectors may be introduced by any method known to the skilled artisan, for example, but without limitation, by transfection, transformation or infection of the host cells, such as, but not limited to, plant cells, mammalian, bacteria, yeast or insect cells.

The term "cloning vector" as used herein, refers to a DNA molecule in which one can integrate a DNA fragment, without loss of replication capacity. Examples of expression vectors are, without limitation, plasmids, cosmids, phage DNA or yeast artificial chromosomes.

The term "expression vector", as used herein, refers to a suitable cloning vector to express a nucleic acid that has been cloned therein after being introduced into a cell, called host cell. Said nucleic acid is generally operably linked to control sequences.

The term "expression" refers to the process by which a polypeptide is synthesized from a polynucleotide and includes transcription of the polynucleotide in a messenger RNA (mRNA) and the translation of such mRNA into a protein or polypeptide. Expression may take place in a host cell.

The term "recombinant vector", as used in the present description refers to a vector suitable for expressing a nucleic acid that has been cloned therein, so that the expression of the peptide is carried out directly in the tissue or target cell. Generally said vector is a virus and is produced by the binding of different nucleic acid fragments from different sources and whose expression results in an infective viral particle composed typically of coat proteins, viral genomes and proteins associated with viral genomes. Expression in a tissue or cell of interest is accomplished by operably linking the polynucleotide to control sequences, specific control sequences, preferably the tissue or cell where it is met; preferably such control sequences are enhancers or promoters. A typical recombinant vector is selected from the group consisting of a lentiviral vector, an adenoviral vector and / or an adeno-associated virus vector.

In a preferred embodiment of the third aspect of the invention the vector is selected from the list that consists of plasmid, bacmid, yeast artificial chromosome, bacteria artificial chromosome, bacteriophage, cosmid, adenovirus, retrovirus, lentivirus or any combinations thereof.

A fourth aspect of the present invention relates to cell that comprises the recombinant cyclic peptide of the first aspect of the invention, the nucleic acid of the second aspect of the invention, and/or the vector of the third aspect of the invention.

The cell can be named as "host cell". The host cells, as it is used in the present invention, relates to any prokaryotic or eukaryotic cell. It refers to a cell wherein the cell comprises the peptide, the polynucleotide, the expression product, the expression cassette or the vector of the invention. To refer to any host cell comprising the polynucleotide of this invention, the term "host cell of the invention" or "host cell of the present invention" can be used. The cell can be a bacteria, a yeast or a fungus. Preferably the cell belongs to the genus Bifidobacterium, Lactococcus, Lactobacillus, Streptococcus, Leuconostoc, Escherichia, Bacillus, Streptomyces, Saccharomyces, Kluyveromyces, Candida, Torula, Torulopsis and Aspergillus. The cell can be also an edible cell, for example: *Lactobacillus acidophilus, Lactobacillus bulgaricus, Lactobacillus casei, Lactobacillus delbrueckii, Lactobacillus fermentum, Lactobacillus gasseri, Lactobacillus paracasei, Lactobacillus johnsonii, Lactobacillus plantarum, Lactobacillus reuteri, Lactobacillus rhamnnosus, or Lactobacillus salivarium.*

The present invention further relates to a cell culture comprising the host cell of the invention. To refer to any cell culture comprising the host cell of the present invention, the term "cell culture of the invention" or "cell culture of the present invention" can be used indistinguishably. The term "cell culture" refers to a cultivation of cells isolated from the same or different type of tissue, or a collection of these cells organized in parts of a plant or in tissues (tissue culture).

A fifth aspect of the present invention relates to pharmaceutical composition that comprises the cyclic peptide of the first aspect of the invention, the nucleic acid of the second aspect of the invention and/or the vector of the third aspect of the invention.

The present invention refers also to a pharmaceutical composition that comprises a mixture of the peptides of the invention.

In the present invention the terms "composition", "pharmaceutical composition", "drug" and "medicament" are used interchangeably.

The term "pharmaceutical composition" herein refers to any substance used for substitution, prevention, diagnosis, alleviation, treatment or cure of diseases in humans or animals. The pharmaceutical composition of the invention can be used either alone or in combination with other pharmaceutical compositions. The term pharmaceutical composition and drug are used in this invention interchangeably. In the context of the present invention relates to a pharmaceutical composition or medicament characterized by comprising the peptide of the invention or the polynucleotide that codifies it allowing their expression in the organism to be treated in a therapeutically effective amount, such that the peptide of the invention performs its function in the target tissue or cell.

A preferred embodiment of the fifth aspect of the present invention refers to the pharmaceutical composition, which additionally comprises an excipient, and/or an acceptable pharmaceutical carrier, and/or an adjuvant, and/or another active principle, or any combinations thereof.

The term "excipient" refers to a substance that helps absorption of the elements of the composition of the invention and actively stabilizes or assists the preparation of the composition in the sense of giving consistency or flavour. Thus, carriers may have the function of holding the ingredients together, such as in the case of starches, sugars or celluloses, sweetening function, the function as a colorant, the protective function of the composition, such as to isolate the air and or moisture, filling the role of a tablet, capsule or other form of presentation, such as is the case of dibasic calcium phosphate, the disintegrating function to facilitate dissolution of the components and its absorption in the intestine, without excluding other excipients not mentioned in this paragraph.

The term "pharmaceutic carrier", refers to a substance used in the pharmaceutical composition or medicament to dilute any component of the present invention included therein to a given volume or weight. The vehicle function is to facilitate the incorporation of other elements, allow better dosage and administration or give substance and form to the composition. When the presentation is liquid, the pharmacologically acceptable carrier is the diluent.

Herein, the term "adjuvant" refers to an agent that increases the effect of the peptide of the invention when given jointly to it or forming part of the same treatment protocol. The pharmaceutically acceptable adjuvants and vehicles that may be used in the pharmaceutical composition of the present invention are vehicles known by those skilled in the art.

In another preferred embodiment the medicament also comprises another active principle, like existing drugs, particularly, the established, conventional co-medication, which are administered to infected patients.

Since most adjuvants are of non-peptide nature, and do not possess the same molecular mechanism or metabolic fate, in principle, no undesired pharmacodynamic drug interactions are thinkable. But synergistic effects can be expected upon combining today's symptomatic pharmacotherapy with the antidote cure of the present invention. Concerning the undesired pharmacokinetic interactions between symptomatic drug therapy or nutrition with the causal antidote cure of the invention, normal care-taking measures have to be taken which are known in the art of clinical medicine, especially parenteral drug administration in presence of peptide agents, like hormones, for instance insulin.

As used herein, the term "active principle" ("active substance", "pharmaceutically active substance", "active ingredient" or "pharmaceutically active ingredient") means any component that potentially provides pharmacological activity or a different effect in the cure, mitigation, treatment, or prevention of disease, or to affect the structure or function of the body of man or other animals.

In another particular embodiment, said pharmaceutical composition is prepared in solid form or aqueous suspension, or in a pharmaceutically acceptable diluent.

The pharmaceutical composition provided by this invention can be administered by any suitable administration route, for which said composition is formulated in the suitable way to the chosen route of administration pharmaceutical form. In a preferred embodiment the medicament for use is presented in an adapted form for oral, parenteral, intramuscular, intraarterial, intravenous, subcutaneous, epidural, intradermic, intraperitoneal or intravesicular administration. In case of wound infection the administration can be done directly into the wound.

A sixth aspect of the present invention relates to the cyclic peptide of the first aspect of the invention, the nucleic acid of the second aspect of the invention or the vector of the third aspect of the invention for use as a medicament.

A seventh aspect of the present invention relates to the cyclic peptide of the first aspect of the invention, the nucleic acid of the second aspect of the invention or the vector of the third aspect of the invention for use in the treatment and/or prevention of a disease caused by a clostridium neurotoxin, preferably the clostridium neurotoxin is selected from the list consisting of: tetanus toxin, botulinum toxin A, botulinum toxin B, botulinum toxin C1, botulinum toxin D, botulinum toxin E, botulinum toxin F, botulinum toxin G, and any combinations thereof.

In a more preferred embodiment of the seventh aspect of the invention the medicament or the cyclic peptide of the first aspect of the invention is administered in a therapeutically effective dose.

The estimated dosage regime of the peptide of the present invention, for an adult person can be estimated by interpolations between one toxicological end point (as known by the expert in the field, for instance by vitamin B12 treatment against cyanid poisoning, assuming a higher susceptibility by 6 orders of magnitude for neurotoxins) and one physiological end point (basal insulin secretion, assuming the same number of cells and cell surface receptor of insulin and NTRs). For example, protection against TeNT or BoNT infection for a total of 1h period can be provided by a combination of bolus injection (5 to 20 µmol/Kg, preferably 10 µmol/Kg) and a steady infusion (1 to 10 µmol/Kg/h, preferably 5 µmol/Kg/h).

The treatment duration will be in days, to "wash out" the neurotoxins by enzymatic cleavage or non-enzymatic hydrolysis of the peptide bonds in the blood stream. Preferably it will be during 1, 2, 3, 4, 5, 6, 7, 8, 9, 10, 11, 12, 13, 14, 15, 16, 17, 18, 19 or 20 days.

The present invention therefore also relates to a method of treatment and/or prevention of a disease caused by a clostridium neurotoxin, preferably the clostridium neurotoxin is selected from the list consisting of: tetanus toxin, botulinum toxin A, botulinum toxin B, botulinum toxin C1, botulinum toxin D, botulinum toxin E, botulinum toxin F, botulinum toxin G, and any combinations thereof; that comprises the administration of a therapeutically effective dose of the peptide of the first aspect of the present invention. The treatment duration will be as explained previously.

Of note, because of its extreme potency clostridium neurotoxins (LD50 approx. 1 to 4 ng for a 1 Kg body weight of a person), even a lethal dose of TeNT may not be sufficient to provoke a response of the human immune system.

Since the antidote is based on a regular fragment of a natural body protein (d5 ectodomain of Trk NTRs), the risk to suffer allergic reaction is minimized for the patients.

In the present invention the term "therapeutically effective dose" (or therapeutically effective amount") refers to the amount of the agent or compound capable of producing a desired effect and will generally be determined by the compounds' characteristics, the route and frequency of administration form thereof and other factors, including age, condition of the patient and the severity of the alteration or disorder.

In a more preferred embodiment in the present invention the treatment or prevention is performed in a human being, man or woman of any age.

In addition multiple sequence alignment (MSA) studies show that the underlying segment of Trk NTRs, which was taken to construct the peptide of the invention, present a very high percent of amino acid sequence similarity. In particular, for this short amino acid segment of animals like pig (*Sus scrofa*), horse (*Equus caballus*), cow (*Bos taurus*), sheep (*Ovis aries musimon*), dog (*Cannis familiaris*), rat (*Rattus norvegicus*) or even chicken (*Gallus gallus*) the percentage of similarityamounts to 100% identity) when compared to the corresponding amino acid sequence segment of human Trk NTRs. Therefore, the same antidote can be used in other animals too. The UniProt identification numbers of the corresponding amino acid sequences of mammalians and bird organisms for the three types of Trk NTRs are given in the following listing: Human: P04629, Q16620 and Q16288; Pig: F1RHK6, P24786 and P24786; Horse: F6YUZ6, F6QPZ0 and F6QD54; Cow: F1MC14, E1BCQ4and E1BCQ4; Sheep: W5P907, W5PGI6 and W5PGI4. Dog: F6XBZ5, E2RKA1 and F1PYP5; Rat: P35739, Q63604 and Q03351; finally Chicken: Q90699, Q91987 and Q91044 for TrkA, TrkB and TrkC NTRs, respectively.

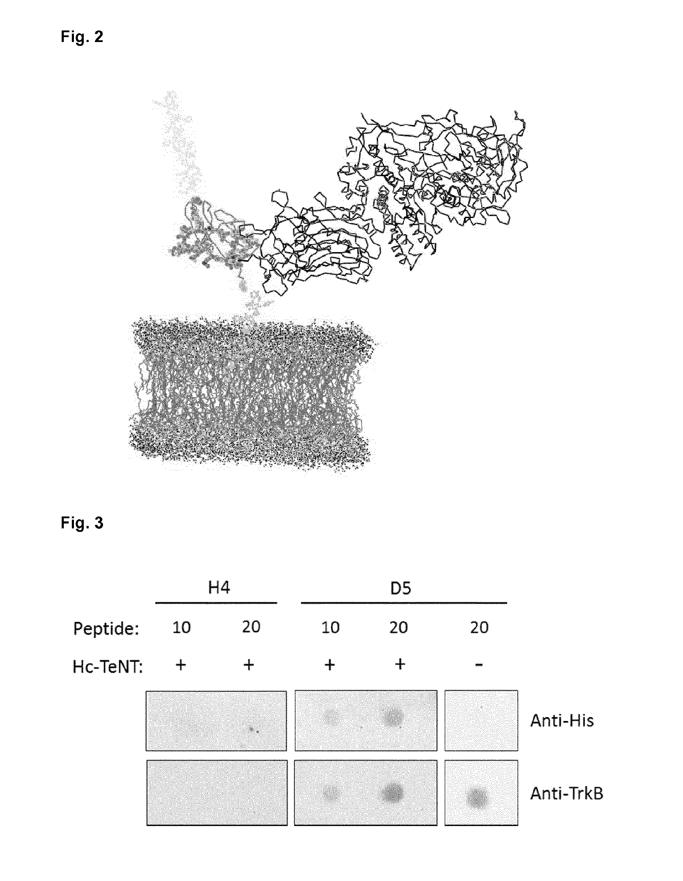

As the expert in the field knows, the human TrkA, TrkB and TrkC are homologous (this means highly conserved). Therefore, the antidote can be used for any of those receptors (see fig. 2).

There are seven different forms of botulinum neurotoxin identified with the letters A to G. Four of these (types A, B, E and occasionally F) cause human botulism. Types C1, D and E cause disease in other mammals, birds and fish. Therefore, the peptide of the present invention is useful in humans, other mammals (for example ruminants such as cattle, sheep or horses) as well as birds, for instance chickens.

The UniProt databank documents the amino acid sequences with the identification numbers for the seven *Clostridium botulinum* subtypes A, B, C1, D, E, F and G neurotoxins. The UniProt identification numbers of the aforementioned seven botulinum neurotoxins are listed in the following: BoNTA: P10845; BoNTB: P10844; BoNTC1: P18640; BoNTD: P19321; BoNTE: Q00496; BoNTF: P30996; andBoNTG: Q60393.

The peptide of the present invention can also be used in food such as canned food or processed food to prevent food intoxication by Clostridium, in a preferred embodiment by *C*. *tetani* or *C*. *botulium* (but also by *C*. *baratii, C. argentinense* or *C*. *butiricum*).

An eighth aspect of the present invention relates to the cyclic peptide of the first aspect of the invention, the nucleic acid of the second aspect of the invention, or the vector of the third aspect of the invention for use as an inhibitor of clostridium neurotoxins; preferably the clostridium neurotoxins are selected from the list consisting of: tetanus toxin, botulinum toxin A, botulinum toxin B, botulinum toxin C1, botulinum toxin D, botulinum toxin E, botulinum toxin F, botulinum toxin G,and any combinations thereof.

The present invention relates to the use of the cyclic peptide of the first aspect of the invention, the nucleic acid of the second aspect of the invention, or the vector of the third aspect of the invention as an inhibitor of clostridium neurotoxins; preferably the clostridium neurotoxins are selected from the list consisting of: tetanus toxin, botulinum toxin A, botulinum toxin B, botulinum toxin C1, botulinum toxin D, botulinum toxin E, botulinum toxin F, botulinum toxin G, and any combinations thereof.

A ninth aspect of the present invention relates to a kit or device which comprises the cyclic peptide of the first aspect of the invention, the nucleic acid of the second aspect of the invention the vector of the third aspect of the invention and/or the cell of the fourth aspect of the invention.

Any of the component of the kit or device can be located on a solid support, for example, but not limited to, glass, plastic, tubes, multi-well plates, membranes, silicon supports, plastic supports, filters, gel layers, metallic supports, any other known support, or a mixture thereof.

A tenth aspect of the present invention relates to the kit or device of the ninth aspect of the invention for use in the treatment or prevention of a disease caused by a clostridium neurotoxin, preferably the clostridium neurotoxin is selected from the list consisting of: tetanus toxin, botulinum toxin A, botulinum toxin B, botulinum toxin C1, botulinum toxin D, botulinum toxin E, botulinum toxin F, botulinum toxin G, and any combinations thereof.

The present invention relates the use of the kit or device of the ninth aspect of the invention in the treatment or prevention of a disease caused by a clostridium neurotoxin, preferably the clostridium neurotoxin is selected from the list consisting of: tetanus toxin, botulinum toxin A, botulinum toxin B, botulinum toxin C1, botulinum toxin D, botulinum toxin E, botulinum toxin F, botulinum toxin G, and any combinations thereof.

A eleventh aspect of the present invention relates to the use of the kit or device of the ninth aspect of the invention as an inhibitor of a clostridium neurotoxin, preferably the clostridium neurotoxin is selected from the list consisting of tetanus toxin, botulinum toxin A, botulinum toxin B, botulinum toxin C1, botulinum toxin D, botulinum toxin E, botulinum toxin F, botulinum toxin G, and any combinations thereof.

The present invention relates to the kit or device of the ninth aspect of the invention for use as an inhibitor of a clostridium neurotoxin, preferably the clostridium neurotoxin is selected from the list consisting of tetanus toxin, botulinum toxin A, botulinum toxin B, botulinum toxin C1, botulinum toxin D, botulinum toxin E, botulinum toxin F, botulinum toxin G, and any combinations thereof.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### BRIEF DESCRIPTION OF THE DRAWINGS

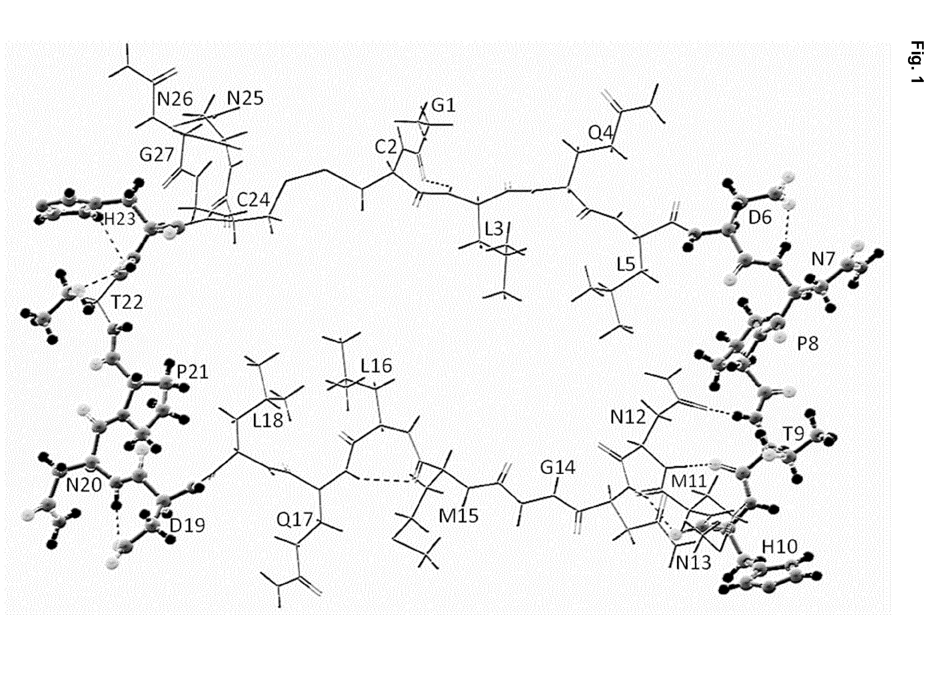

Figure 1. Model display of the antidote compound of the invention with residue labels. The 27 residue-long cyclic polypeptide is ring-closed through a disulfide bridge (yellow S-S-bond, upper mid-section). Hydrophobic side chains are packed into the ring center. The reading is clockwise (G1 at 12 o' clock (h); D6 to the right at 3h; H10 to the bottom at 6h; N19 to the left at 9h; and G27 at 11 h). The outwards residues are interacting with the C-terminal parts of Clostridial neurotoxins (fragment C or Hc, exactly the Hcc parts). The inwards residues form a hydrophobic core and stabilize the conformation, L3, L5 with P8 (right), or L16, L18 and P21 (left). The First half1 (ball&stick display, right) and the Second half2 (ball&stick display, left) have identical amino acid sequences (DNPTH). Either only one part binds to a monomeric Trk receptor, or in case of a lipid raft homo- or heterodimerization of two identical or non-identical Trk complex, both sides can bind simultaneously.
Figure 2. Scenic display to illustrate the molecular mechanism of action. A Trk NTR is symbolically visible (leftmost vertically stretched linear molecule which is anchored in the membrane block). Its structurally known d5 ectodomain is a spherical protein domain (leftmost, just above the membrane block). A complete neurotoxin macromolecule (rightmost, largest structure) is shown in its liganded pose to the d5 ectodomain of the Trk NTR. In ball&stick representation the cyclic antidote of the invention is visible in superposition to d5 to display its pose where it replaces the neurotoxin binding to d5 of Trk NTR. Of note, a computed superposition between ectodomain d5 and d5ring is displayed here. Albeit in reality either a (d5 ectodomain of) Trk NTR molecule or (!) an antidote molecule (d5ring, d5 peptide) is bound to the neurotoxin macromolecule (rightmost). Both ligands (d5, d5ring) are in direct competition to a common receptor (C-terminal part of heavy chain of the Clostridial neurotoxins). This simulated finding was experimentally confirmed.
Figure 3. Hc-TeNT binds *in vitro* to a synthetic cyclic peptide of the invention based on TrkB d5 ectodomain. This so-called d5 peptide is attached to a nitrocellulose membrane, and then membrane is blocked and incubated with Hc-TeNT (10 ng/ml). The dot-blot is analyzed using anti-TrkB antibody and anti poly-Histidine (anti-His). Controls without Hc-TeNT or without d5 peptide are analyzed in parallel.

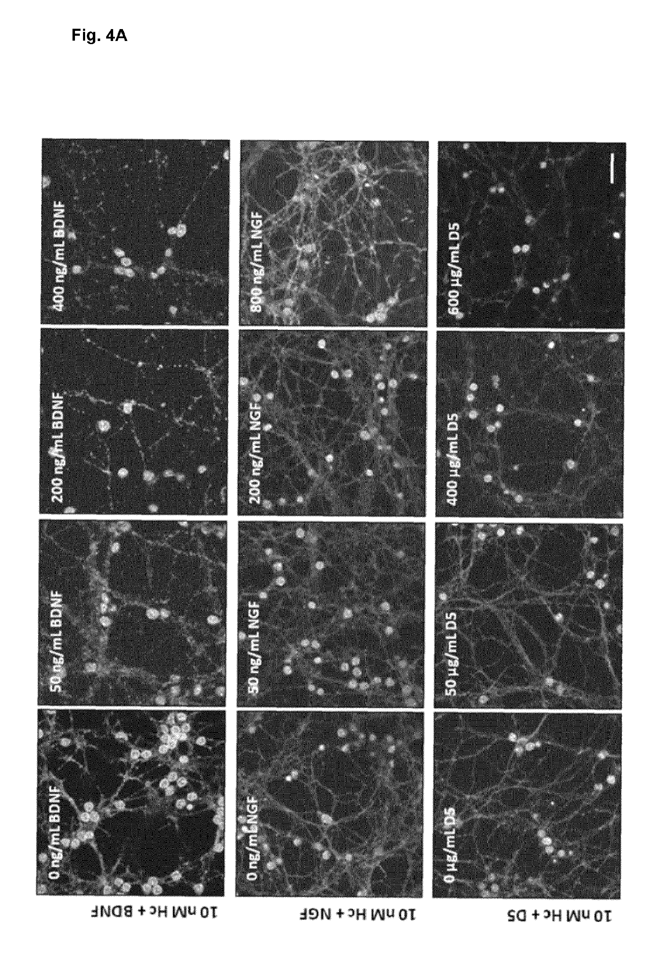

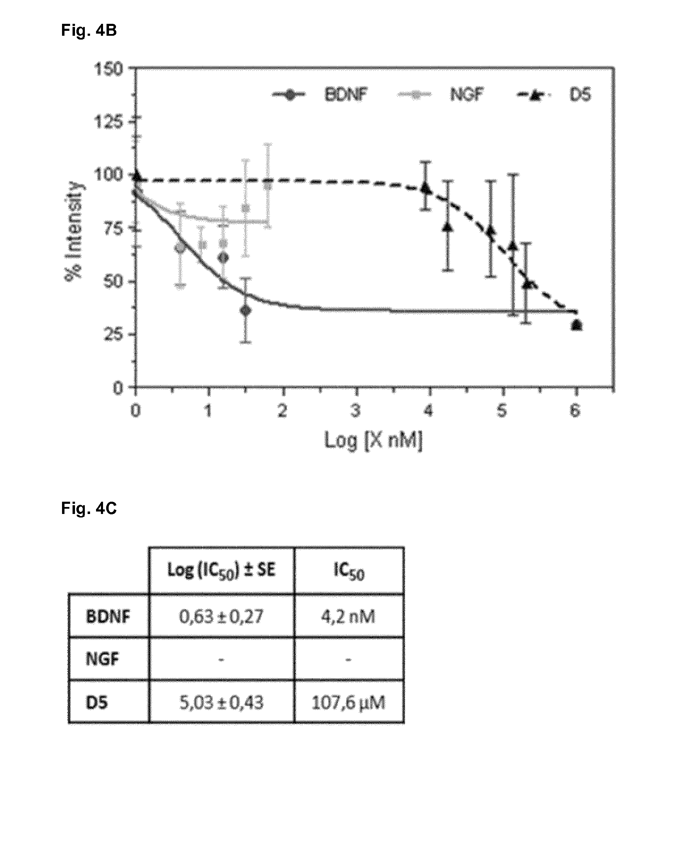

Figure 4. BDNF and d5 peptide, but no NGF, competes with Hc-TeNT for the binding to cerebellar granule neurons. **(A)** Cells were treated for 30 min at 4°C with 10 nMHc-TeNT labeled with AlexaFluor555, and adding increasing amounts of BDNF, NGF or d5 peptide. Cells were fixed and nucleus were stained with DAPI. Images were taken with a confocal microscope and analyzed to measure the mean fluorescence intensity of red channel. (Scale bar 20µm.) **(B)** Resulting binding competition curves are shown in the graph (BDNF competition, NGF and d5). **(C)** Table with inhibition constants calculated with GraphPad Prism from the graph.

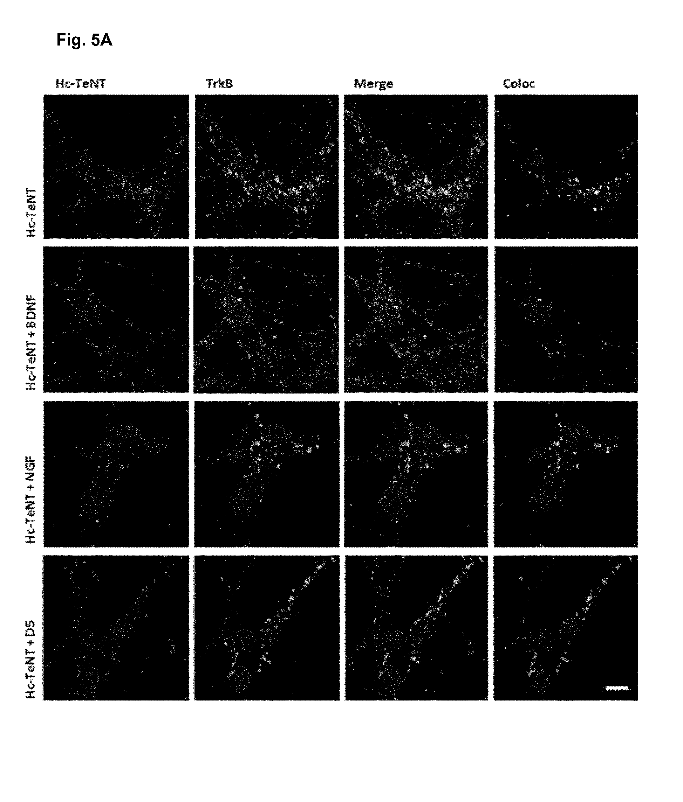

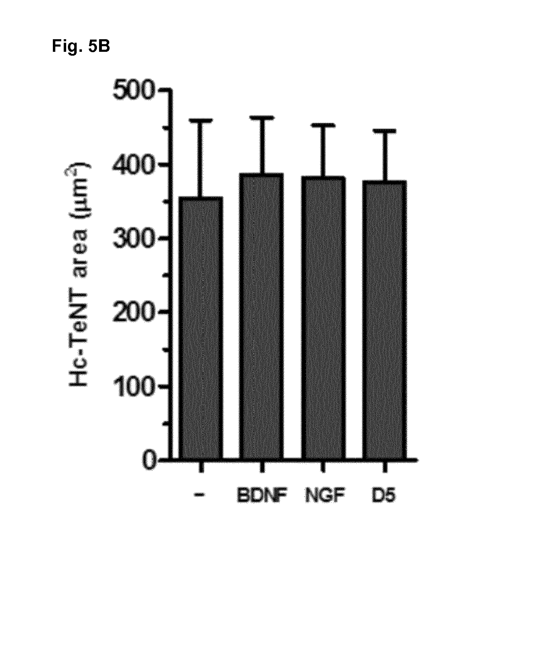

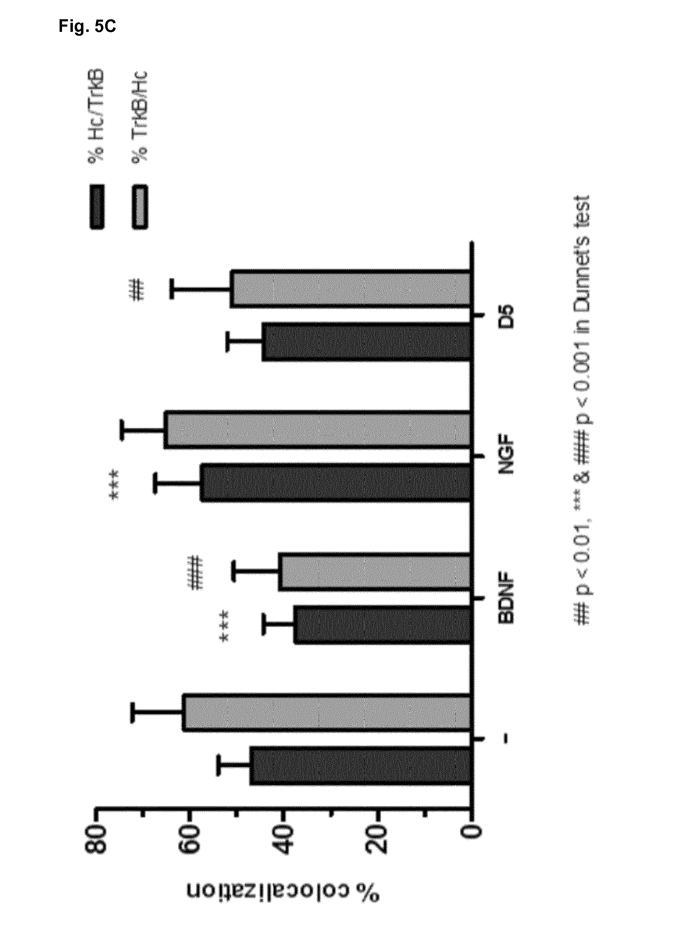

Figure 5.Internalization competition between Hc-TeNT and BDNF, NGF and d5 peptide. **(A)** CGN were incubated with Hc-TeNT labelled with AlexaFluor555 (20 nM) for 1 h at 37°C (in red). An antibody against TrkB was incubated at the same time. In some conditions 20 µMd5, 200 ng/mL BDNF or 200 ng/mL NGF were added. After this time, cells were fixed and an immunostaining was performed with a secondary antibody AlexaFluor488 against anti-TrkB. Images were taken with a confocal microscope and analysed with Imaris software. Colocalized points were shown in white. (Scale bar = 5 µm.) **(B)** Red channel fluorescence occupied area for each condition. **(C)** Colocalization percentage between red channel (Hc-TeNT) and green channel (TrkB).

### EXAMPLES

### Example 1.In silico experiments:

### Materials and Methods

Molecular models were generated using the general-purpose software toolbox Vega ZZ [Pedretti *et al.,* 2004] and Swiss PDB Viewer [Guex and Peitsch, 1997]. Various bioinformatics tools at two web sites were consulted [Kanz *et al.,* 2005; Gasteiger *et al.,* 2003], for instance the Blast search engine (Basic Local Alignment Search Tool) found similar amino acid sequences [Altschul *et al.,* 1997] or the links to UniProt online services allowed the downloading of amino acid sequences and inspection of protein annotation [Dimmer *et al.,* 2012]. Related crystal structures were retrieved at the PDB site [Berman *et al.,* 2000] and checked out by What_check [Hooft *et al*., 1996] or licensed software suites: What_if [Vriend, 1990], while molecular dynamics simulations were conducted using the Yasara program [Krieger *et al*., 2002]. Multiple sequence alignment (MSA) studies were interpreted with respect to conserved residues and homologies [Larkin *et al.,* 2007]. Homology based protein modeling was carried out to generate a complete structure of the TeNT molecule based on the known crystal structure of the Hc-fragment and the crystal structure of the 3D template BoNT. A more comprehensive description of protein homology modeling has been presented elsewhere [Scior *et al.,* 2007].

### Detailed Description of the Results

### Molecular description of the clostridial neurotoxins, BoNT and TeNT.

The TeNT like the BoNT molecules form a dichain molecule with a disulfide bridge. The light chain of BoNT (∼50 kD - amino acids 1-448) acts as a zinc (Zn2+) endopeptidase similar to tetanus toxin with proteolytic activity located at the N-terminal end. The heavy chain (∼100 kD - amino acids 449-1280) provides cholinergic specificity and is responsible for binding the toxin to presynaptic receptors; it also promotes light-chain translocation across the endosomal membrane.

The carboxyl-terminal half of the Hc-fragment (TeNT Hcc) provides for cellular receptor and ganglioside binding activities and shows a β-trefoil shape [Umland *et al.,* 1997; Knapp *et al.,* 1998]. Specific binder residues of the β trefoil domain were discussed in the literature [Emsley, P, 2000]. In particular, two ganglioside-binding sites were reported: the lactose-binding pocket "W" located around tryptophan W1289 and adjacent residues like histidine H1271 and D1222, and the sialic acid-binding pocket "R" around arginine R1226 and adjacent residues like tyrosine1229 [Chen *et al.,* 2009; Emsley *et al.,* 2000; Rummel *et al.,* 2003; Jayaraman *et al.,* 2005]. Rummel *et al.* reported on carbohydrate-binding sites of Hcc which constitute a prerequisite for tetanus toxicity [Rummel *et al.,* 2003]. The Hc-fragment assists motor nerve cell recognition, binding and internalization (cell uptake) via endocytosis. The process takes place in this highly specific fashion at synapses of cholinergic motor neurons. The holotoxin is retrogradely transported within the motor neuron axons toward the spinal cord (retrograde axonal transport). Quite intriguing, but amenable to drug targeting & delivering development, the isolated Hc-fragment is fully capable of binding and intraaxonal transport. Prior to blocking inhibitory motor neurons (tetanic spasm), TeNT is released into the synaptic clefts, then bound and taken up again, this time by inhibitory motor neurons [Calvo *et al.,* 2012].

### The membrane receptors for the neurotoxin cell uptake

The core part of the invention was the working hypothesis of Trk NTR as targets for invading Clostridial neurotoxins, then followed by the computed model identification of certain residues in the ectodomain d5 of Trk A, TrkB and TrkC NTR on the membrane surface of human cells to target and bind to invading Clostridial neurotoxins (BoNT and TeNT). With our *in silico* study we could identify certain amino acids of the Trk NTRs as targets of amino acids of BoNT as well as TeNT which were hitherto unknown for their target recognition and binding functions **(****figures 1** **and** **2****).** This novel finding was then confirmed by biochemical *in vitro* experiments

In contrast to our finding (novelty), the published literature has established the molecular mechanism for the neurotoxin internalization (cell uptake) through binding to ganglioside receptors on the cell surface.

The relevant issue for the antidote design is the recognition of a hitherto unknown binding between Trk and the Hcc part of clostridial neurotoxins. This finding is really unexpected if one follows the current stream documented by the published literature, which establishes the gangliosides as binding receptors for incoming neurotoxins prior to cell uptake, and subsequent, cell release inhibition and finally death of the organism.

Based on our mutational bindings studies combined with bioinformatics as well as molecular modeling grounds we present convincing evidence for the involvement of the aforementioned neurotrophin binding ectodomain (d5) of TrkA, TrkB or TrkC receptors (see figures 1 to 5). We present the specificity of the neurotrophin cell surface receptors as a target protein prior to BoNT and TeNT cell-uptake, all of which explains the extremely high toxicity despite the observed low binding affinities via gangliosides in the outer leaflet of neuronal cell membranes. Binding to ectodomain d5 as a further mechanistic component for cell recognition and cell uptake can also explain the contradicting bioactivities of apparently equivalent *in vivo* and *in vitro* bioassays, some of them prepared from neuronal tissue. Previously an additional protein receptor has been proposed to account for the activity differences, but it has never been found. The peptide of the invention, now, gives the answer, since it binds to the TeNT and BoNT, and it constitutes a fragment of the ectodomain d5 of Trk NTRs (the 14 amino acid-long segment called "First Half1"), it demonstrates that Trk NTRs are the target biomolecules for invading TeNT and BoNT, and not just gangliosides as the functional receptors for TeNT and BoNT.

The role for gangliosides as receptors for tetanus neurotoxin was experimentally observed by molecular binding assays [Holmgren *et al.,* 1980; Chen *et al.,* 2009]. Before our postulation of a Trk receptor component, theoretical implications raised the question whether gangliosides constitute the sole binders for tetanus toxin and botulinum neurotoxin [Chen *et al.,* 2009; Binz and Rummel, 2009].

The human neuronal cell surfaces are rich in gangliosides. They show, however, low affinities to the Hc domains of BoNTs and thereby cannot explain the extraordinary neurotoxicity in nanomolar range (reviewed in [Swaminathan, 2011]. Moreover, the binding is sensitive to proteases suggesting the existence of protein receptor involvement. Further inconsistencies are low and high affinity sites in brain tissues and the drop in the binding affinity between *in vivo* and *in vitro* bioassays. Various binding modes incorporating one or two distinct ganglioside contacts as well as a two-receptor binding model with protein receptor involvement have been formulated (reviewed in [Binz and Rummel, 2009]).

Recently, the hitherto known facts about the binding mechanism(s) were reviewed [Calvo *et al.,* 2012]. The established view implies a dual receptor mechanism for the binding of the Clostridial neurotoxins to neurons which includes only ganglioside-binding components [Montecucco, 1986].

Nevertheless, the relevance of either gangliosides or protein receptors for the uptake process is subject to ongoing research work. Current research results, however, cannot discard that other structures like surface proteins could act as another, chemically distinct interaction site to better explain the extremely high affinity and specificity and not just with mere carbohydrate-binding sites [Binz and Rummel, 2009; Calvo *et al.,* 2012].

### Biochemical description of the mechanism of action: the neurotoxicity

On a biochemical level, toxicity takes place via a four step process: (i) surface accumulation and binding to cell membranes of target neurons and (ii) uptake into neuronal cells, (iii) translocation, and finally, (iv) cell damage through enzymatic activities. But the toxins sort out and act differently: BoNTs in peripheral tissues whereas TeNT on central nerve system - despite their common overall-structures comprised of light and heavy chains and their binding to polysialo-gangliosides which were first reported for TeNT as early as 1961 [Calvo *et al.,* 2012].

### Example2. Molecular description of the BoNT and TeNT antidote

The proposed antidote is a polypeptide which constitutes the binding-relevant fragment to the NTR. It is elsewhere called the 14 residue-long segment "First Half1" of the peptide of the invention. It is therefore unlikely that it leads to allergic reactions since it is an amino acid sequence which is totally conserved throughout the species. The other half of the peptide of the invention which is elsewhere called the 13 amino acid long segment "Second Half2" is a copy of the "First Half1" with only two reciprocally mutated amino acids: one methionine was changed to cysteine and an already existing cysteine back into methionine [Larkin *et al.,* 2007]. In particular, the GCL motif in the C-Terminal part was mutated to GML, while the HMNNG motif was changed into HCNNG, found in the second half of the cyclic antidote. Hence, the cyclic antidote is composed of two almost identical halves of an original segment of the human target receptor NTR (TrkB).

### Molecular description and chemical data of the antidote.

The cyclic structure is a polypeptide composed of 27 residues (SEQ ID NO: 3, wherein the cycle is located between the position 2 and 24 between the two cysteines). Concerning the stereochemistry, all amino acids belong to the natural proteogenic L-series, according to the Fischer projection, i.e. the chirality at the carbon C alpha atoms is as follows: the two cysteines show (R) configuration while all other amino acids show (S) according to the Cahn-Ingold-Prelog priority rules.

**Table 1.Listing of chemical data and properties for the antidote molecule:**

| | |
|---|---|
| Atoms | : 386 |
| Heavy atoms | : 202 |
| Residues | : 27 |
| Formula | : C118H184N39O41S4 |
| Molecular weight | : 2933.220 Daltons |
| Monoisotopic mass | : 2931.239474 Daltons |
| Gyration radius | : 12.5330 Å |
| Geometry center | : -13.7581 -2.3150 16.3348 |
| Mass center | : -13.9066 -2.3187 16.3029 |
| Appx. dimensions | : 32.9101 27.3050 29.8950 |
| Total charge | : -1.0000 |
| Dipole | : 157.6723 Debye |
| Surf. area (1.40) | : 3433.9 Å² (ds=33.1 A) |
| Polar area (PSA) | : 1738.4 Å² (apolar=1695.5 Å²) |
| Volume | : 2511.3 Å³ (dv=16.9 A) |
| Ovality | : 3.4089 |
| logP (Crippen) | : -14.7625 |
| Lipole (Crippen) | : 1.0916 |
| LogP (Broto) | : -35.4670 |
| Lipole (Broto) | : 1.5133 |
| Virtual logP | : -12.5920 |
| Molar refractivity | : 687.2466 |
| Predicted charge | : -1 |
| (composition of charged fragments) N-Terminal -NH3+ | |
| Aminoacidic charge | : -2 (aminoacid composition) |
| Solubility | : water soluble |

**Table 2. Listing of the amino acidic composition:**

| Residue | | times % | added Mass % | |
|---|---|---|---|---|
| Asn | 6 | 22.22 | 684.616 | 23.34 |
| Asp | 2 | 7.41 | 228.159 | 7.78 |
| Cys | 2 | 7.41 | 204.270 | 6.96 |
| Gln | 2 | 7.41 | 256.258 | 8.74 |
| Gly | 3 | 11.11 | 174.178 | 5.94 |
| His | 2 | 7.41 | 274.278 | 9.35 |
| Leu | 4 | 14.81 | 452.630 | 15.43 |
| Met | 2 | 7.41 | 262.392 | 8.95 |
| Pro | 2 | 7.41 | 194.230 | 6.62 |
| Thr | 2 | 7.41 | 202.208 | 6.89 |

**Table 3. Listing of the amino acids engaged in intramolecular hydrogen bonding and the overall estimated potential energy gain in [Kcal/mol].**

| | |
|---|---|
| H-bond acceptors: | 43 |
| H-bond donors: | 47 |
| Number of H-bonds: | 15 |
| H-bond potential energy gain: | -4.3 |

BoNTs and TeNT act through its carboxyl-terminal part (Hcc, or acronym HCR/T in the literature) Fragment C (Hc) in modeling. The extracellular domain of TrkB d5 is considered as the receptor for BoNTs as well as TeNT or its peptide Hc. To inhibit the activity of BoNTs or TeNT the mechanism is based on mimicing the affinity of BoNTs or TeNT (via their Hcc parts or Hc = Fragment C) to the d5 TrkB receptor domain, thereby replacing the active part of TrkB domain d5, by the novel "scavanger" molecule which constitutes the antidote "d5ring", sometimes called d5ring in the computed studies, or "peptide D5" mainly in the experimental studies. In a more general view, it is a competitive biochemical reaction between the Trk receptors and the novel antidote for engaging the C-terminal part of BoNTs or TeNT.

### Example 3: Parenteral antidote administration.

The parenteral antidote is administered directly in the infected wound, blood stream (i.v.), or capillary zone of infected tissues (s.c., i.m., etc.) according to the time frame left in the infection progress.

The indication is for patients without active tetanus vaccination or immune compromised patients, or - in cases of doubts - as "ultima ratio" since infections are live-threatening events which make hospitalization and intensive care mandatory.

The polypeptide ring is closed by a disulfide bridge (see figure 1).

### Example 4: Molecular description of the antidote action mechanism

The computer simulations showed that neurotoxins (BoNT and TeNT) bind to the same cell membrane receptors which are the physiologic target of neurotrophins. With its function as a "false" receptor binding site, imitating the "correct" binding site for neurotoxins (the Trk NTRs), the antidote of the invention prevents the incoming neurotoxins from binding to these human cell membrane receptors (Trk NTRs) and the intoxication is effectively stopped at this early stage (see Figure 1).

Following this argumentation the polypeptidic antidote is a target receptor-mimetic, i.e. it imitates (or mimics) the d5 domain of human Trk receptors (NTRs). It can also be seen as a suicide substance which is given to sacrifice itself and be taken and bound to the toxins instead of the cell surface Trk receptors. Its suicidal function is to bind to the free botulinum or tetanus toxins in blood plasma which prevents free neurotoxins to gain access to the cells upon cell membrane binding and uptake - and thusly protecting the organism from toxic damage by tetanus infection. For this reason it can be referred to as the suicide protein or polypeptide.

In a less canonical way it also parallels the function of a scavenger molecule. A scavenger molecule is a chemically defined substance which is added to prevent other chemical compounds to react to unwanted reaction products, e.g. the antioxidants vitamin C (ascorbic acid) or vitamin E (tocopherol congeners) are scavengers of oxygen or free radicals in the liquid media.

Moreover, it can be seen as a sort of reverse or anti-decoy. Conventionally, decoys are molecules, which should not bind to a target thusly leaving it without a biological response or activity. Such non-binders: are used to validate ligand docking programs or for benchmarking studies. The attribute "reverse" here means that the contrary of this holds true here: namely, it binds.

In our novel computer simulations neurotoxins (BoNT, TeNT) show high affinity to the cell membrane receptor of neurotrophins. All hitherto known neurotrophins (NTs) have selectivities - in a way that each of them prefers a certain Trk receptor subtype in lower doses over the other subtypes. But they are not totally specific because, even if a given NT binds to a certain Trk receptor, it is not capable of excluding other NTs to bind, too [Pattarawarapan and Burgess, 2003]. Specificity of each neurotrophin corresponds to nonconserved amino acids in the primary structure among the neurotrophins while others - conserved ones - could be involved in shared receptor-binding in analogous regions on their surfaces [Reichardt, 2006].

### The neurotrophin receptors as template for the antidote structure:

The present *in silico* approach studies the molecular interaction zone between BoNTs and TeNT ligand and their possible target regions (hitherto called the interaction patch) on the nerve cell surface. The models are based on crystallographic data: the observed structure of the TeNT Hc-fragment [Knapp *et al.,* 1998] and the ligand-receptor binding domain complexes between NGF and TrkA [Robertson *et al.,* 2001] as well as NT4/5 and TrkB [Banfield *et al.,* 2001].

The known ganglioside recognition mode was incorporated into our model of TeNT and BoNTs with the homologous Trk receptor family.

Crystal structures of several neurotrophin ligands (NGF, NT-3, NT-4/5), their respective binding domains on receptors like TrkA, TrkB, TrkC or p75^{NTR}, as well as the hitherto known TeNT Hc-fragments were retrieved: 1A8D [Knapp *et al.,* 1998]; 1AF9 [Umland *et al.,* 1997], 1HCF [Banfield *et al.,* 2001], 1WWA [Ultsch *et al.,* 1999], 1WWB [Ultsch *et al.,* 1999], 1WWC [Ultsch *et al.,* 1999], and 1WWW [Ultsch *et al.,* 1999]. The holoprotein sequence of TeNT was also used in our MSA studies (UniProtKB identification number: P04958).

Multiple sequence alignment (MSA) studies show an absence of homology between NTs and TeNT Hcc. An identity score of 20% is far too low for a typical 130-residue length. Actually, at this length it should be above 30% to ensure structural similarity in order to safely reflect phylogenetically related proteins [Scior *et al.,* 2007]. To this regard the following inspected ligand complexes lent valuable structural insight: 3HN1 [Chen *et al.,* 2009], 3HMY [Chen *et al.,* 2009], 1D0H [Emsley *et al.,* 2000], 1YXW [Jayaraman *et al.,* 2005], 1YYN [Jayaraman *et al.,* 2005]. In addition to the discovered residues belonging to the known ganglioside-binding sites [Chen *et al.,* 2009; Emsley *et al.,* 2000; Rummel *et al.,* 2003; Jayaraman *et al.,* 2005], we succeeded in identifying others which define the interaction patch of the NTR interface on the β-trefoil domain: Glu264 (E1127), Tyr266 (Y1129), Lys311 (K1174), Ser338 (S1201), Asn341 (N204), Glu343 (E1206), Glu447 (E1310), and Asp452 (D1315). This novel binding interface was modeled on analogy assumptions in absence of any homology. MSA analyses were also conducted to compare the varying degrees of conservation among the binding residues on neurotrophic receptors (NTRs).

Computer-based 3D modeling simulates the antidote of the invention in action. The antidote molecule replaces the binding to the d5 domain of Trk cell membrane surface receptor family. A scenic illustration is presented in figure 2 - although the complete structure of a Trk NTR remains unknown for vast parts of the extracellular segments, its bilayer membrane segment as well as its intracellular parts. Nevertheless, its binding to TeNT can be simulated because the three-dimensional structures of both, ectodomain d5 and Hc domain of TeNT or BoNTs, were elucidated by crystallography.

The calculated non-bonded interactions between residues of ligands and the neurotrophin receptors (NTRs) are listed in details (see table 3 and table 4). In the table 4 each row lists the interaction between Trk NTR amino acids (called Receptor) with others from TeNT (last column). Upon comparison to the observed interaction pattern - published with the PDB entries 1WWA, 1WWB and 1WWC [Ultsch *et al.,* 1999] - all computed interactions taken together lead to the conclusion that TeNT or BoNTs are stronger binders than neurotrophins while TeNT binds stronger to TrkB and less to TrkC or TrkA, for instance. Particularly, the TeNT - TrkB interface has an excess of non-bonded attraction over the observed neurotrophin complex (NT4/5): six hydrogen bonds and two polar ones through basic histidine side chains - or alternatively - two ion bridges through the imidazolium ring of protonated histidine. The theoretical implications are twofold: on the one side TeNT should be the better binder toward TrkB, while - on the other side - TeNT appears to be an even stronger binder when compared to the endogenous neurotrophin (NT) ligands with subnanomolar activities. In living organisms, the holotoxin successfully competes for neurotrophic nerve cell receptors at even lower concentrations and accommodates head-on (Hcc) on the cell surface. In view of evolutionary adaptation, death can be effectively imposed to the host vertebrate organism creating the necessary anaerobic environment for massive bacterial reproduction before continuing a new infection cycle through soil contamination from the cadaver.

**Table 4. Listing of the calculated non-bonded interactions of binding residues between the Trk receptors and the neurotoxin ligand. The following symbols are used: (w, p) Hb = (water-mediated, polar) Hydrogen-bond; +- = cation - anion bridge; no = no observation; ap = nonpolar hydrophobic; bb = backbone.**

| Interface | Receptor | Ligand |
|---|---|---|
| Computed non-bonded interaction for columns 2&3 | NT-binding domain, d5 of NTR : TrkA/B/C | Computed complex with Hcc of TeNT |
| Hb/Hb/Hb | T325/S327/S345 | N883 |
| no/+-/+- | S326/K328/K346 | E1127 |
| no/+-/+- | | E1206 |
| no/Hb/no | F327/Y329/I347 | S1201 |
| no/Hb/no | | E1206 |
| pHb/+-/pHb | N349/D349/N366 | K1174 |
| no/no/+- | Q350/N350/K367 | E1127 |
| Hb/Hb/pHb | | Y1129 |
| pHb/pHb/no | | K1174 |
| pHb or +-/pHb or +-/+- | H297/H299/R316* (* and not adjacent L315 or H319) | E1310 |
| no/pHb or +-/ pHb or +- | L333/H335/Y353* (* and not adjacent Y352, H349) | D1315 |

### Interpretation of the computed binding simulations.

The modeler (inventor) devised this computational study primarily from crystallographic data [Berman *et al.,* 2000] of the TrkB d5 binding domain andneurotrophin ligands (NGF, NT-3, NT-4/5), their respective binding domains (TrkA, TrkB, TrkC) as well as the TeNTHc-fragments, 1HCF [Banfield *et al.,* 2001], 1WWA [Ultsch *et al.,* 1999], 1WWB [Ultsch *et al.,* 1999], 1WWC [Ultsch *et al.,* 1999], and 1 WWW [Ultsch *et al.,* 1999].The overall structure of neurotoxins is known thanks to the crystal structure representing the holotoxin of BoNT type B (1EPW) [Swaminathan andEswaramoorthy, 2000] in addition to various structures such as 1A8D [Knapp *et al.,* 1998]; 1AF9 [Umland *et al.,* 1997], also known as C-fragment of TeNT with or without co-crystallized ganglioside receptor fragments bound to two carbohydrate-binding pockets in close vicinity to the proposed Trk-binding patch (1FV2, 3HMYand 3HN1) [Fotinou *et al.,* 2001; Chen *et al.,* 2009; Knapp *et al.,* 1998].

All three target-biomolecules were crystallographically described, some with co-crystallized neurotrophic ligands. The three TrkA, TrkB and TrkC fold into the same overall structure because the percentage sequence identities safely lie above the 100-residue threshold of 30 % (see table 5).

**Table 5. Percentage sequence identities of the ligand binding domain d5 of all three Trk NTR.**

| It can be seen that TrkB is 44 % identical with either TrkA (first row) or TrkC (last row). Comparing Trk A toTrkC the identity score drops to 38 percent. | | | | | | |
|---|---|---|---|---|---|---|
| PDB_chain | Ligand | Length | PDB_chain | Ligand | Length | % id |
| 1WWA_X | d5TrkA | 101 | 1WWB_X | d5TrkB | 103 | 44 |
| 1WWA_X | d5TrkA | 101 | 1WWC_A | d5TrkC | 118 | 38 |
| 1WWB_X | d5TrkB | 101 | 1WWC_A | d5TrkC | 118 | 44 |

Missing parts of the neurotoxin 3D-models can be obtained by modeling techniques called homology protein modeling, since the clostridial family is closely related and their structures are likely to be very similar [Scior *et al.,* 2007]. Precisely, BoNT and TeNTs share significant sequence homology what raises likelihood up to an almost-certainty for sharing a common ancestral protein fold. This finding supports the proposed common Trk target for the neurotoxins (see table 5). All neurotrophins (NTs) are very similar with homology percentages between 50 % and 56 %. They are all known Trk receptor binders. Homology, however, does not exist (but functional convergence in evolutionary times) between clostridial neurotoxins and the human protein family of neurotrophins, since their scores fall lower than the empiric threshold of approx. 30 %. According to traditional thinking, two sequences are practically unrelated in structure and function if their length and percentage sequence homologies drop below a given threshold. Yet, neurotrophin dimerization resulting in similar size and folding to neurotoxins laid the groundwork for the analogy modeling approach between them (see table 6).

**Table 6. Primary structure analyses of proposed and known d5 binders. The neurotrophins were either modelled as homodimers or taken from dimeric crystal complexes: (NT-3)₂ [Gong et al., 2008], (NT-4)₂ [Robinson et al., 1999], (NT-4/5)₂ [Banfield et al., 2001], (beta-NGF)₂ [He and Garcia, 2004] or (NT4+BDNF) [Robinson et al., 1999]. The percentage sequence homology scores (Gonnet matrix) [Larkin et al., 2007] are listed in the second last column and refer to the Hcc of serotype B (BoNT) as reference sequence (100 %). TeNT encodes the Hcc of tetanus toxin. Abbr.: aa: amino acid(s); #: number; q: charge; Gravy: Grand average of hydropathicity; hom.: homology [Gasteiger et al., 2003].**

| Ligand | # of aa | Net q | Aliphatic index | Gravy | Mol. Mass [Da] | Mol. Vol. [Å³] | Hom. Score % | Threshold % |
|---|---|---|---|---|---|---|---|---|
| BoNT | 211 | +3 | 85 | -0.6 | 25838 | 23145 | 100 | > 25 |
| TeNT | 213 | +3 | 69 | -0.9 | 25632 | 22861 | 28 | > 25 |
| (BDNF)₂ | 218 | +14 | 62 | -0.6 | 24615 | 22010 | 7 | > 30 |
| (NGF)₂ | 202 | +7 | 67 | -0.3 | 22705 | 20161 | 6 | > 30 |
| (NT-3)₂ | 180 | +2 | 66 | -0.8 | 20732 | 18393 | 5 | > 30 |
| (NT-4)₂ | 216 | +9 | 68 | -0.4 | 23130 | 20522 | 3 | > 28 |

### Experimental proofs of principle

During 2012, 2013 José Aguilera *et al.* at UAB, realized mutational studies according to the results of the computational design study of 2011, 2012 by inventor Thomas Scior. The Mutational studies were successfully carried out as proof of concept in which amino acids where changed to show that they play a role at the binding site between bacterial toxins and the cell surface receptor prior to cell uptake.

### Example 5: role of trkb neurotrophin receptor as a high affinity protein receptor for tetanus toxin

### Interaction between Hc-TeNT and TrkB

In this work we have analyzed the interaction between Hc-TeNT and TrkB receptor using a docking study to determine the regions implicated in the union. We have found that Hc-TeNT is capable of binding a TrkB-derived peptide of the predicted interacting region *in vitro.* Moreover treatment with Hc-TeNT in cultured neurons causes phosphorylation of the TrkB receptor - suggesting an interaction between the two molecules - whereas the mutation of three residues in Hc-TeNT sequence (Hc-Mut) attenuates this effect. We also have analyzed the specific binding and subsequent internalization of both the Hc-TeNT as well as the mutated fragment in cultured neurons by confocal microscopy and flow cytometry, finding in all cases a loss of affinity of the mutated domain (see figures 1 to 5).

The determination of the TeNT receptor would be a great advance in drug delivering research dealing with the peripheral nerve system to the CNS.

### CONCLUSIONS

TeNT mimics the natural binders to that extend that when the neurotoxic (L-TeNT) domain is cut apart the reminder (isolated Hc-TeNT) becomes a true neurotrophin analog -even lacking any homology between their sequences. In particular, our analog protein modeling shows the neurotrophin-binding interface underwent functional convergence during biological evolution to strongly bind to the d5 ectodomain of TrkB. Moreover-since evolutionary convergence emerges upon the necessity of shared function but is based on random mutational events - it is clear that receptors' binding specificities and affinities cannot be expected to be the same between the neurotoxins and neurotrophins. The latter are the endogenous high-affinity Trk binders that act as neural-growth-factor of vertebrates in the form of homo- or hetero-dimer, highly basic polypeptidic neurotrophins (NT-3, NT-4/5, BDNF, GDNF, or NGF).

### CLONING AND EXPRESSION OF Hc-Mut (Y266A, K311A, E343A).

It was agreed to do point mutations in the amino acid sequence of Fragment C (Hc-TeNT) to check whether it were possible to weaken the Trk-receptors affinity, specifically the TrkB receptor and to know what part of the TeNT interacted as ligand with the Trk receptors. The aforementioned reduction in TrkB activity would also confirm the mode of action of the TeNT, all of which leads us to the idea that the d5 peptide binds to TrkB replacing Hc-TeNT.

A construct of 905 pb consisting of a C-terminal segment of Hc-TeNT was synthesized to introduce the suggested mutations. This fragment was cloned in the pMK vector (Life Technologies: Paisley, UK) using SacII and Kpnlrestriction sites, thus obtaining the plasmid pMK_Hc (3205 pb, kanamycin resistance). This construct contains the segment extending from base 431 to 1221 of the Hc sequence, including several interspersed repeats of the same sequence that allow for the inclusion in the same construct the selected amino-acidic positions in both wild-type and mutated forms. At the end of each repeat, different restriction sites were introduced that delimited each of the wild-type and mutant residues, in order to allow us to obtain single, double or triple mutants. To introduce these restriction sites some conservative nucleotide changes have been made in the sequence, so that no mutation was incorporated in the amino acid sequence.

To obtain the triple mutant Hc-Mut (Y266A, K311A, E343A), pMKHc-TeNT was digested with Sal I and then relegated to remove the fragment containing K1174, and thereafter the plasmid was digested with MluI and relegated to remove E1206. Finally, the resulting plasmid was digested with SacII and KpnI and the digestion product was cloned in the pYN1 vector using the KpnI and SacII restriction sites to yield the pYN1-Mutexpression vector. The introduction of the correct mutations was evaluated by DNA sequencing of the recombinant clones. The recombinant plasmid was transformed into *E*. *coli* BL21 (Qiagen; Chatsworth, California, USA), and the expression of the mutated protein was performed in the same way as described in the previous section.

### Final conclusions:

The mutant protein (Hc-Mut) was tested in a cerebellar granule neurons model and showed a decrease in the TrkB activation when compared with the activation caused by Hc-TeNT wild type - thus indicating the probable implication of this position in the interaction. These results were found in excellent agreement with the molecular modeling studies that had been conducted prior to in vitro experiments. Albeit the final data interpretation concluded that more residues could be involved in such protein-protein interactions.

In principle, however, the interaction between Hc-TeNT and TrkB receptor-predicted in the molecular docking simulations - were confirmed by the Hc-TeNT and the TrkB-derived peptide union observed in the dot-blot assay. Phosphorylation of the TrkB receptor caused by the treatment with Hc-TeNT also indicated an interaction among the two proteins, as well as the competition with BDNF for the binding to the receptor.

Mutation in three residues (Y266A, K311A, E343A) of Hc-TeNT caused a reduction in TrkB activation which was accompanied by an affinity loss for the receptor corroborated in binding and uptake assays.

In the same manner immunochemistry experiments showed the colocalization between Hc-TeNT and TrkB in CGN cells both in binding and internalization conditions. Mutations in Hc-TeNT sequence resulted in a decrease of TrKB colocalization in both conditions.

The determination of the TeNT receptor will be a major breakthrough for the discovery of a gateway leading from the peripheral to the central nervous system. This will have important implications in designing new drugs capable of crossing the blood-brain barrier.

What is more relevant in the present work: novel polypeptides designed according to their attachment points within the binding domains of the neurotrophic receptors (TrkA, TrkB, TrkC) which will be capable of forming neutralizing associates (complexes) to the Clostridial neurotoxins (TeNT and BoNT) may become the first specific drugs for the causal treatment of tetanus disease and botulism in the near future.

TrkB was taken as a starting point for its postulated affinity to Hc-TeNT during modeling. Then, we designed a cyclic peptide of the invention (SEQ ID NO: 3) with an amino acid sequence length of 27amino acids fourteen of which reflect a segment of the neurotrophin-binding domain d5 of TrkB. The novel cyclic peptide of the invention is also referred to as "d5ring" or "d5 peptide".

Applying bioinformatics, it was verified that all three targets, TrkA, TrkB and TrkC, possess highly conserved amino acid sequences in the corresponding sequence to ensure high affinity. When compared to the 14 residue long segment of TrkB (100%) which is the "First Half" of the peptide of the invention (SEQ ID NO:1), the corresponding TrkA and TrkC segments show a 86 % and 79 % homology, respectively.

In the antidote of the present invention the cyclic peptide allows to "freeze in" a reduced range of conformations around the desired bioactive conformations excluding unproductive conformational changes into conformations which will not bind to target or bind to undesired biomolecules as a side effect. This leads to favorable enthalpy contributions for the exergonic Free energies of binding.

The ring form "freezes in" relevant conformations around the active conformations. Subsequently, the entropy of the system is still high enough, to ensure favorable thermodynamic properties to yield negative Free energies of binding. In theory an open (linear) form of the peptide of the invention will favor entropy even more but an open form of the peptide of the invention will not adopt an active conformation in the organism to get hold of the invading neurotoxic proteins (in statistical mechanics known as "random walk"). Under *in vivo* conditions the open form even risks to get "nodded", twisted, or even cleaved by proteases or hydrolases which can recognize, fragments or unfold certain substrate segments to be hydrolyzed. Especially exo-peptidases recognize lose ends of fragments for cleavage at the N-term or C-term of peptides. All this is avoided by cyclization of the peptide of the invention. It is an essential feature for binding TeNT and BoNT.

The ring form gives the right orientation to the amino acids. The hydrophobic ones among them orient inwards while the TeNT and BoNT-binding amino acids face the outside of the ring. Their side chains form the binding interface (see figure 1, table 3 and table 4).

The ring form also enhances the mobility and solubility, as it is a fact in the art known to Medicinal chemistry experts, illustrated by peptide-based antibiotics.

The ring form helps to assure and validate the successful synthesis adding amino acids in the correct order.

In case of homo- or heterodimerization of the natural receptors, the NTRs, the cyclic form with the "bifacial" two (almost identical) halves should also have a positive impact - on performing a sort of "imitation" or mimic of that receptor multimerization step.

We took a test peptide with a scrambled order of the amino acids, as a negative control. The sequence of the scrambled peptide was the following: SAPATGGVKKPHRYRPG (SEQ ID NO: 4), with an Hydrogen atom as the N-terminal group and with a Amide group as the C-terminal group.

We extended the range of application by adding more interpretations to our *in silico* models.

The binding segment (called "FIRST HALF1") of the peptide of the invention is the same, regardless of the species. We documented that human, pig, horse, cow, sheep, dog, rat and chicken have the same segment of their ectodomain d5 of their respective Trk NTR.

Minor, homologous amino acid differences are seen, not between species, but between TrkA, TrkB and TrkC isoforms. Within the same species, the three NTRs are almost identical in the "First Half1" segment of those 14 amino acids which constitute the cyclic peptide antidote of the invention. Hence, in order to enhance the antidote potential, there is only a minor need of individual antidotes for each Trk NTR. This observation could lead to the idea of having all three segments (of TrkA, TrkB and TrkC) mixed in the antidote of this invention - and the antidote preparation would have all three segments as cyclic peptides in one formulation together. Of note, such mixtures would be still valid for all documented species(human, pig, horse, cow, sheep, dog, rat and chicken have the same).

We extended the applicability domain of the TeNT experiments to be valid also for BoNT, because we could show that all 7 subtypes of BoNT, A through G, have the same or homologous amino acids in their binding patches (regions). With few binding amino acids the TeNT and BoNT are recognized and bond by the antidote of the invention (or a "three cyclic peptide" mixture).

### Example 6: Hc-TeNT fragment binds the cyclic peptide homologous to d5 domain of TrkB receptor of the present invention.

Molecular modeling revealed that the Hc-TeNT fragment and the d5 extracellular domain of TrkB receptor can neatly dock and that this strong interaction is stabilized by the formation of several hydrogen bonds and ion bridges (see table 3 and table 4). According to this *in silico* model, a 27 amino acid-long cyclic peptide (d5 peptide of the invention) (cyclic SEQ ID NO: 3 with the disulfide bridge) was synthesized. It is homologous to the d5 domain of Trk NTRs (d5 in short). A dot-blot assay was used to test the ability of a recombinant Hc-TeNT fragment to bind the cyclic d5 peptide of the invention. In this assay the cyclic d5 peptide was first attached to a nitrocellulose membrane (10 µg and 20 µg of peptide), and it was then blocked and incubed with a recombinant Hc-TeNT fragment. After that we revealed the membrane using an antibody against poly-histidines that recognized the six histidine residues in the C-terminal of the recombinant Hc-TeNT. A polyclonal antibody against TrkB was used to detect the d5 peptide attached to the membrane. We could also observe that Hc-TeNT was able to bind specifically to the d5 peptide, but it could not be detected in a control with a random-sequence peptide (referred as H4 peptide) with the same number of residues as the d5 peptide. Controls without d5 peptide or without Hc-TeNT fragment were performed to assure antibody specificity. The experiments confirm the molecular modeling study and corroborate that Hc-TeNT is able to bind the d5 peptide in *in vitro* experiments (see figure 3).

In the competition curves (see figure 5) we could observe how BDNF (as well as d5 peptide were capable to compete with the Hc-TeNT fragment for binding to cell membrane. Both of them displaced the Hc-TeNT bound to the membrane when increasing amounts of BDNF and d5 peptide were added. In contrast, treatments with NGF seemed to have no effect on the quantity of membrane-bound Hc-TeNT, or at least at the assayed concentrations. The IC₅₀ values for each molecule were obtained from the competition curves. The IC₅₀ reflected the concentration of unlabeled competitor necessary to displace 50% of specifically bound Hc-TeNT. The higher the affinity of the competitor for the receptor the lower the calculated IC₅₀, since a lower concentration of the competitor will be needed to displace the molecule of interest. BDNF showed a calculated IC₅₀ of 4.2 nM (60 ng/mL), while IC₅₀ value for the d5 peptide is 107.6 µM (317.3 µg/mL). The IC₅₀ for NGF could not be calculated since we could not adjust a competition curve for this compound.

BDNF is a well-known natural agonist of TrkB and exhibits a high dissociation constant (K_{D}) for its receptor: about 1nM-10pM, according to the technique used to determine it [Soppet *et al.* 1991; Dechant *et al.* 1993; Urfer *et al.* 1995; Windisch *et al.* 1995]. The fact that BDNF is able to displace the binding of Hc-TeNT to the cell membrane indicates that both molecules compete for the same binding site on the receptor. In granule cerebellar neurons (a TrkB⁺, TrkA⁻, p75^{NTR+} cellular model), BDNF is mostly interacting with TrkB, but also, albeit with less affinity, with p75^{NTR}. If Hc-TeNT were interacting with p75^{NTR}, the added NGF would be able to also displace its binding to the membrane. However, this effect is not observed - so,Hc-TeNT and BDNF are competing mainly for the binding site in TrkB receptor in this model. Based on the previous dot-blot assay, the cyclic d5 peptide should bind and block the binding site in Hc-TeNT- thusly preventing its interaction with endogenous TrkB receptor. Binding competition assays corroborate this assumption since d5 peptide displaces membrane-bound Hc-TeNT-although less effective than BDNF. The fact that such high concentrations of d5 peptide are required for displacement of the Hc-TeNT binding, may indicate that other regions not blocked with d5 peptide and involved in binding could exist in Hc-TeNT, or maybe that d5 peptide is not large enough to block the entire interaction zone. In any case, the concentration of d5 peptide necessary for the displacement is in a feasible range for a drug.

### BDNF can displace the interaction between Hc-TeNT and TrkB receptor in endocytic vesicles.

As described in the previous section, both BDNF and d5 peptide, but not NGF, are capable to compete with Hc-TeNT for the same receptor in the cell membrane. This suggests that by reducing its binding to the receptor, the Hc-TeNT internalization into the cells could be affected, too. Internalization competition assays were performed in granule cerebellar neurons using Hc-TeNT fragment labeled with AlexaFluor555 (20 nM) and adding BDNF, NGF or d5 peptide in a high concentration (30 nM, 30nM and 20µM, respectively). Cells were incubated with the different compounds for 1 h at 37°C, as well as with an antibody against TrkB receptor expressed in the membrane. The d5 peptide was pre-incubated with Hc-TeNT for 1h previous to the addition in the culture. After incubation, cells were washed with an acidic solution to eliminate signal from the remaining membrane-bound non-internalized Hc-TeNT. Then an immunofluorescence was performed using a secondary antibody labeled with AlexaFluor488 against primary TrkB antibody and staining the nucleus with DAPI. Cells were analyzed in a confocal microscope and representative images are shown in figure 6A The area occupied by Hc-TeNT was calculated and is shown in figure 6B. In this case, no changes in the amount of Hc-TeNT endocyted between control condition and conditions with competitors were detected. The addition of BDNF, NGF or d5 peptide seems not to affect the internalization of Hc-TeNT fragment under these conditions.

In view of these results we considered whether the interaction between Hc-TeNT and TrkB could be affected by the addition of the competitors. Under these conditions Hc-TeNT could be entering in the cell in the same amount but through a different mechanism independent from TrkB. We calculated the co-localization percentage of Hc-TeNT and TrkB in these cells incubated with Hc-TeNT and competitors. Co-localized pixels are shown in figure 5A while calculated percentages are indicated in figure 5C. It was already shown that in cerebellar granule neurons (brain granule neurons) treated with Hc-TeNT-AlexaFluor555, about the 47% of Hc-TeNT were colocalized with TrkB in the same endocytic vesicles. The addition of BDNF caused a significant decrease in the amount of Hc-TeNT associated with TrkB in vesicles, about 37% co-localization. In parallel, the percentage of TrkB in co-localization with Hc-TeNT was also diminished (∼40% colocalization in contrast to 61% under control conditions). Most probably added BDNF could bind to TrkB and then both were internalized together, displacing Hc-TeNT from the endocytic vesicles. In stark contrast, the addition of NGF caused an increased amount of Hc-TeNT overlapping with TrkB (57% co-localization approximately). The fact that the addition of NGF promoted the interaction between TeNT-Hc and TrkB could be explained if the Hc-TeNT, like other neurotrophins, may interact with more than one neurotrophin receptor. Thus, the NGF could displace the interaction between Hc-TeNT and other receptors and increase the amount of toxin available to interact with TrkB. In this cellular model NGF can only interact with p75^{NTR}. It was described that Hc-TeNT and p75^{NTR} receptor shared endocytic vesicles in cultured motor neurons and that both molecules were able to interact and cause the activation of different signaling pathways [Deinhardt 2006b, Cubí *et al.* 2013]. If the added NGF is interacting with p75^{NTR} within the cells, it could displace a hypothetical union between Hc-TeNT and p75^{NTR}, thus explaining the increasing amount of Hc-TeNT in co-localization with TrkB. Finally, no differences in percentage of Hc-TeNT in colocalization withTrkB were observed when adding the d5 peptide to the cells in comparison with control condition, about 44% co-localization in d5-containing conditions and 47% in control. In contrast, the percentage of TrkB overlapping with Hc-TeNT is affected by the addition of the peptide, decreasing significantly the percentage from 61% in control to 51% in presence of d5 peptide. In binding competition assays d5 peptide could displace the Hc-TeNT fragment. However, we could not see this effect in internalization assays. One explanation could be that higher concentration of peptide is needed to observe a reduction in the Hc-TeNT/TrkB co-localization percentage. Another possibility is that other regions in Hc-TeNT outside the d5 peptide binding site play a role during internalization. Hence it cannot be ruled out that a larger peptide of the d5-TrkB domain had a greater effect.

### REFERENCES:

Altschul, S.F., T.L. Madden, A.A. Schäffer, J. Zhang, Z. Zhang, W. Miller, and D.J. Lipman. 1997. Gapped BLAST and PSI-BLAST: A new generation of protein database search programs. Nucleic Acids Res. 25:3389-3402.
Banfield, M.J., R.L. Naylor, A.G.S. Robertson, S.J. Allen, D. Dawbarn, and R.L. Brady. 2001. Specificity in Trk receptor:neurotrophin interactions: The crystal structure of TrkB-d5 in complex with neurotrophin-4/5. Structure. 9:1191-1199. doi:10.1016/S0969-2126(01)00681-5.
Berman, H.M., J. Westbrook, Z. Feng, G. Gilliland, T.N. Bhat, H. Weissig, I.N. Shindyalov, and P.E. Bourne. 2000. The Protein Data Bank. Nucleic Acids Res. 28:235-242.
Binz, T., and A. Rummel. 2009. Cell entry strategy of clostridial neurotoxins. J. Neurochem. 109:1584-1595. doi:10.1111/j.1471-4159.2009.06093.x.
Brook I. (2007) Infant botulism. Journal of Perinatology. 27: 175-80.
Calvo, A.C., S. Oliván, R. Manzano, P. Zaragoza, J. Aguilera, and R. Osta. 2012. Fragment C of tetanus toxin: New insights into its neuronal signaling pathway. Int. J. Mol. Sci. 13:6883-6901. doi:10.3390/ijms13066883.
Chen, C., Z. Fu, J.J.P. Kim, J.T. Barbieri, and M.R. Baldwin. 2009. Gangliosides as high affinity receptors for tetanus neurotoxin. J. Biol. Chem. 284:26569-26577. doi:10.1074/jbc.M109.027391.
Cubí, R., A. Candalija, A. Ortega, C. Gil, and J. Aguilera. 2013. Tetanus Toxin Hc Fragment Induces the Formation of Ceramide Platforms and Protects Neuronal Cells against Oxidative Stress. PLoS One. 8*.* doi:10.1371/journal.pone.0068055.
Cui Y, Zhang C, Wang Y, Shi J, Zhang L, Ding Z, Qu X, Cui H. (2012) Class Ilabacteriocins: diversity and new developments. Int. J. Mol. Sci., 13: 16668-707.
Dechant, G., S. Biffo, H. Okazawa, R. Kolbeck, J. Pottgiesser, and Y. a Barde. 1993. Expression and binding characteristics of the BDNF receptor chick trkB. Development. 119:545-558.
Deinhardt, K., S. Salinas, C. Verastegui, R. Watson, D. Worth, S. Hanrahan, C. Bucci, and G. Schiavo. 2006. Rab5 and Rab7 Control Endocytic Sorting along the Axonal Retrograde Transport Pathway. Neuron. 52:293-305. doi:10.1016/j.neuron.2006.08.018.
Dimmer, E.C., R.P. Huntley, Y. Alam-Faruque, T. Sawford, C. O'Donovan, M.J. Martin, B. Bely, P. Browne, W.M. Chan, R. Eberhardt, M. Gardner, K. Laiho, D. Legge, M. Magrane, K. Pichler, D. Poggioli, H. Sehra, A. Auchincloss, K. Axelsen, M.C. Blatter, E. Boutet, S. Braconi-Quintaje, L. Breuza, A. Bridge, E. Coudert, A. Estreicher, L. Famiglietti, S. Ferro-Rojas, M. Feuermann, A. Gos, N. Gruaz-Gumowski, U. Hinz, C. Hulo, J. James, S. Jimenez, F. Jungo, G. Keller, P. Lemercier, D. Lieberherr, P. Masson, M. Moinat, I. Pedruzzi, S. Poux, C. Rivoire, B. Roechert, M. Schneider, A. Stutz, S. Sundaram, M. Tognolli, L. Bougueleret, G. Argoud-Puy, I. Cusin, P. Duek-Roggli, I. Xenarios, and R. Apweiler. 2012. The UniProt-GO Annotation database in 2011. Nucleic Acids Res. 40.
Emsley, P., C. Fotinou, I. Black, N.F. Fairweather, I.G. Charles, C. Watts, E. Hewitt, and N.W. Isaacs. 2000. The structures of the H(C) fragment of tetanus toxin with carbohydrate subunit complexes provide insight into ganglioside binding. J. Biol. Chem. 275:8889-8894. doi:10.1074/jbc.275.12.8889.
Fotinou, C., P. Emsley, I. Black, H. Ando, H. Ishida, M. Kiso, K. a. Sinha, N.F. Fairweather, and N.W. Isaacs. 2001. The Crystal Structure of Tetanus Toxin Hc Fragment Complexed with a Synthetic GT1b Analogue Suggests Crosslinking between Ganglioside Receptors and the Toxin. J. Biol. Chem. 276:32274-32281. doi:10.1074/jbc.M103285200.
Gasteiger, E., A. Gattiker, C. Hoogland, I. Ivanyi, R.D. Appel, and A. Bairoch. 2003. ExPASy: The proteomics server for in-depth protein knowledge and analysis. Nucleic Acids Res. 31:3784-3788.
Gill, D.M. 1982. Bacterial toxins: a table of lethal amounts. Microbiol. Rev. 46:86-94.
Gong, Y., P. Cao, H. Yu, and T. Jiang. 2008. Crystal structure of the neurotrophin-3 and p75NTR symmetrical complex. Nature. 454:789-793.
Guex, N., and M.C. Peitsch. 1997. SWISS-MODEL and the Swiss-PdbViewer: An environment for comparative protein modeling. Electrophoresis. 18:2714-2723.
Hassel B (2011) Tetanus: Pathophysiology, Treatment, and the Possibility of Using Botulinum Toxin against Tetanus-Induced Rigidity and Spasms. Toxins, 5: 73-83.
Hassel, B. 2013. Tetanus: Pathophysiology, treatment, and the possibility of using botulinum toxin against tetanus-induced rigidity and spasms. Toxins (Basel). 5:73-83. doi:10.3390/toxins5010073.
He, X.-L., and K.C. Garcia. 2004. Structure of nerve growth factor complexed with the shared neurotrophin receptor p75. Science. 304:870-875. doi:10.1126/science. 095190.
Holmgren, J., H. Elwing, P. Fredman, and L. Svennerholm. 1980. Polystyrene-adsorbed gangliosides for investigation of the structure of the tetanus-toxin receptor. Eur. J. Biochem. 106:371-379.
Hooft, R.W., G. Vriend, C. Sander, and E.E. Abola. 1996. Errors in protein structures. Nature. 381:272.
Jayaraman, S., S. Eswaramoorthy, D. Kumaran, and S. Swaminathan. 2005. Common binding site for disialyllactose and tri-peptide in C-fragment of tetanus neurotoxin. Proteins Struct. Funct. Genet. 61:288-295. doi:10.1002/prot.20595.
Kanz, C., P. Aldebert, N. Althorpe, W. Baker, A. Baldwin, K. Bates, P. Browne, A. van den Broek, M. Castro, G. Cochrane, K. Duggan, R. Eberhardt, N. Faruque, J. Gamble, F. Garcia Diez, N. Harte, T. Kulikova, Q. Lin, V. Lombard, R. Lopez, R. Mancuso, M. McHale, F. Nardone, V. Silventoinen, S. Sobhany, P. Stoehr, M.A. Tuli, K. Tzouvara, R. Vaughan, D. Wu, W. Zhu, and R. Apweiler. 2005. The EMBL nucleotide sequence database. Nucleic Acids Res. 33.
Knapp, M., B. Segelke, and B. Rupp. 1998. The 1.61 Angstrom Structure of the Tetanus Toxin Ganglioside Binding Region: Solved by MAD and Mir Phase Combination. In Am.Cryst.Assoc., Abstr.Papers (Annual Meeting). 25,90.
Krieger, E., G. Koraimann, and G. Vriend. 2002. Increasing the precision of comparative models with YASARA NOVA - A self-parameterizing force field. Proteins Struct. Funct. Genet. 47:393-402.
Larkin, M.A., G. Blackshields, N.P. Brown, R. Chenna, P.A. Mcgettigan, H. McWilliam, F. Valentin, I.M. Wallace, A. Wilm, R. Lopez, J.D. Thompson, T.J. Gibson, and D.G. Higgins. 2007. Clustal W and Clustal X version 2.0. Bioinformatics. 23:2947-2948.
Montecucco, C. 1986. How do tetanus and botulinum toxins bind to neuronal membranes? Trends Biochem. Sci. 11:314-317.
Pattarawarapan, M., and K. Burgess. 2003. Molecular Basis of Neurotrophin - Receptor Interactions. J. Med. Chemitry. 46:5277-5291.
Pedretti, A., L. Villa, and G. Vistoli. 2004. VEGA - An open platform to develop chemo-bio-informatics applications, using plug-in architecture and script programming. J. Comput. Aided. Mol. Des. 18:167-173.
Reichardt, L.F. 2006. Neurotrophin-regulated signalling pathways. Philos. Trans. R. Soc. Lond. B. Biol. Sci. 361:1545-1564. doi:10.1098/rstb.2006.1894.
Robertson, a G., M.J. Banfield, S.J. Allen, J. a Dando, G.G. Mason, S.J. Tyler, G.S. Bennett, S.D. Brain, a R. Clarke, R.L. Naylor, G.K. Wilcock, R.L. Brady, and D. Dawbarn. 2001. Identification and structure of the nerve growth factor binding site on TrkA. Biochem. Biophys. Res. Commun. 282:131-141. doi:10.1006/bbrc.2001.4462.
Robinson, R.C., C. Radziejewski, G. Spraggon, J. Greenwald, M.R. Kostura, L.D. Burtnick, D.I. Stuart, S. Choe, and E.Y. Jones. 1999. The structures of the neurotrophin 4 homodimer and the brain-derived neurotrophic factor/neurotrophin 4 heterodimer reveal a common Trk-binding site. Protein Sci. 8:2589-2597. doi:10.1110/ps.8.12.2589.
Rummel, A., S. Bade, J. Alves, H. Bigalke, and T. Binz. 2003. Two carbohydrate binding sites in the HCC-domain of tetanus neurotoxin are required for toxicity. J. Mol. Biol. 326:835-847. doi:10.1016/S0022-2836(02)01403-1.
Shapiro RL, Hathaway C, Swerdlow DL (1998) Botulism in the United States: a clinical and epidemiologic review. Ann. Intern. Med., 129: 221-228.
Scior T, and Wahab HA (2007) Structure prediction of proteins with very low homology: a comprehensive introduction and a case study on aminopeptidase. in Book title: Drug Design Research Perspectives. Nova Science Publishers. Editor: Stanley P. Kaplan. ISBN 978-1-60021-823-1. 675-708.
Smith LA, and Rusnak JM (2007) Botulinum neurotoxin vaccines: past present, and future. Crit. Rev. Immunol., 27: 303-318.
Soppet, D., E. Escandon, J. Maragos, D.S. Middlemas, S.W. Reid, J. Blair, L.E. Burton, B.R. Stanton, D.R. Kaplan, T. Hunter, K. Nikolics, and L.F. Parada. 1991. The neurotrophic factors brain-derived neurotrophic factor and neurotrophin-3 are ligands for the trkB tyrosine kinase receptor. Cell. 65: 895-903.
Supuran CT (2012) Inhibition of bacterial carbonic anhidrases and zinc proteases: from orphan targets to innovative new antibiotic drugs. Curr. Med. Chem., 19: 831-44.
Swaminathan, S. 2011. Molecular structures and functional relationships in clostridial neurotoxins. FEBS J. 278:4467-4485.
Swaminathan, S., and S. Eswaramoorthy. 2000. Structural analysis of the catalytic and binding sites of Clostridium botulinum neurotoxin B. Nat. Struct. Biol. 7:693-699.
Ultsch, M.H., C. Wiesmann, L.C. Simmons, J. Henrich, M. Yang, D. Reilly, S.H. Bass, and a M. de Vos. 1999. Crystal structures of the neurotrophin-binding domain of TrkA, TrkB and TrkC. J. Mol. Biol. 290:149-159. doi:10.1006/jmbi.1999.2816.
Umland, T.C., L.M. Wingert, S. Swaminathan, W.F. Furey, J.J. Schmidt, and M. Sax. 1997. Structure of the receptor binding fragment HC of tetanus neurotoxin. Nat. Struct. Biol. 4:788-792.
Urfer, R., P. Tsoulfas, L. O'Connell, D.L. Shelton, L.F. Parada, and L.G. Presta. 1995. An immunoglobulin-like domain determines the specificity of neurotrophin receptors. EMBO J. 14:2795-2805.
Vriend, G. 1990. WHAT IF: a molecular modeling and drug design program. J. Mol. Graph. 8:52-56.
Wilson BA, and Ho M (2014) Cargo-delivery platforms for targeted delivery of inhibitor cargos against botulism. Curr. Top Med. Chem., 14: 2081-83.
Windisch, J.M., B. Auer, R. Marksteiner, M.E. Lang, and R. Schneider. 1995. Specific neurotrophin binding to leucine-rich motif peptides of TrkA and TrkB. FEBS Lett. 374:125-129. doi:10.1016/0014-5793(95)01047-I.
Zhang, Y., Buchko, G.W., Qin, L., Robinson, H., Varnum, S.M. 2011, Cristal structure of the receptor binding domain of the botulinum C-D mosaic neurotoxin reveals roles of lysines 1118 and 1136 in membrane interactions. Biochem. Biophys. Res. Commun. 404:407-12.

## Claims

1. A cyclic peptide that comprises an amino acid sequence of at least 70%, 80%, 85%, 90%, 95%, 96%, 97%, 98%, 99% or 100% identity with SEQ ID NO: 1, preferably the peptide is SEQ ID NO: 1.

2. The cyclic peptide according to claim 1 which also comprises at least another amino acid sequence of at least 70% identity with SEQ ID NO: 1, preferably at least one amino acid sequence of sequence SEQ ID NO: 2.

3. The cyclic peptide according to claims 1 or 2 comprising, preferably consisting of, sequence SEQ ID NO: 3.

4. The cyclic peptide according to any one of claims 1 to 3 which is cycled between at least two cysteine amino acids forming a disulfide bridge, preferably when the cyclic peptide is SEQ ID NO: 3 the disulfide bridge is located between the position 2 and 24.

5. A nucleic acid that codifies for the cyclic peptide according to any one of claims 1 to 4.

6. A vector that comprises the nucleic acid according to claim 5.

7. A cell that comprises the cyclic peptide according to any one of claims 1 to 4, the nucleic acid according to claim 5 and/or the vector according to claim 6.

8. A pharmaceutical composition that comprises the cyclic peptide according to any one of claims 1 to 4, the nucleic acid according to claim 5 and/or the vector according to claim 6.

9. The pharmaceutical composition according to claim 8 which additionally comprises an excipient and/or an acceptable pharmaceutical carrier and/or an adjuvant, and/or another active principle, or any combinations thereof.

10. The cyclic peptide according to any one of claims 1 to 4, the nucleic acid according to claim 5 or the vector according to claim 6 for use as a medicament.

11. The cyclic peptide according to any one of claims 1 to 4, the nucleic acid according to claim 5, or the vector according to claim 6 for use in the treatment and/or prevention of a disease caused by a clostridium neurotoxin, preferably the clostridium neurotoxin is selected from the list consisting of: tetanus toxin, botulinum toxin A, botulinum toxin B, botulinum toxin C1, botulinum toxin D, botulinum toxin E, botulinum toxin F, botulinum toxin G, and any combinations thereof.

12. The cyclic peptide according to any one of claims 1 to 4, the nucleic acid according to claim 5 or the vector according to claim 6 for use as an inhibitor of a clostridium neurotoxin, preferably the clostridium neurotoxin is selected from the list consisting of: tetanus toxin, botulinum toxin A, botulinum toxin B, botulinum toxin C1, botulinum toxin D, botulinum toxin E, botulinum toxin F, botulinum toxin G, and any combinations thereof.

13. A kit which comprises the cyclic peptide according to any one of claims 1 to 4, the nucleic acid according to claim 5, the vector according to claim 6 and/or the cell according to claim 7.

14. The kit according to claim 13 for use in the treatment and/or prevention of a disease caused by a clostridium neurotoxin, preferably the clostridium neurotoxin is selected from the list consisting of: tetanus toxin, botulinum toxin A, botulinum toxin B, botulinum toxin C1, botulinum toxin D, botulinum toxin E, botulinum toxin F, botulinum toxin G, and any combinations thereof.

15. The kit according to claim 14 for use as an inhibitor of a clostridium neurotoxin, preferably the clostridium neurotoxin is selected from the list consisting of: tetanus toxin, botulinum toxin A, botulinum toxin B, botulinum toxin C1, botulinum toxin D, botulinum toxin E, botulinum toxin F, botulinum toxin G, and any combinations thereof.
